(19) Europäisches Patentamt
European Patent Office
Office européen des brevets

(11) Publication number: **0 019 369**
**B1**

(12) # EUROPEAN PATENT SPECIFICATION

(45) Date of publication of patent specification: **07.03.84**

(21) Application number: **80301289.7**

(22) Date of filing: **22.04.80**

(51) Int. Cl.³: **C 07 D 209/48,**
C 07 D 401/12,
C 07 D 417/12,
C 07 D 317/66,
A 01 N 47/36

(54) **Isothioureido isoindolediones, process for their preparation and use as plant regulators.**

(30) Priority: **03.05.79 US 35875**

(43) Date of publication of application:
**26.11.80 Bulletin 80/24**

(45) Publication of the grant of the patent:
**07.03.84 Bulletin 84/10**

(84) Designated Contracting States:
**DE IT SE**

(56) References cited:
**FR - A - 2 154 390**
**FR - A - 2 309 602**

(73) Proprietor: **Gulf Oil Corporation**
**P. O. Box 1166**
**Pittsburgh Pennsylvania 15230 (US)**

(72) Inventor: **Kirkpatrick, Joel Lee**
**17 Canal Run East**
**Washington Crossing Pennsylvania 18977 (US)**
Inventor: **Patel, Natu Raojibhai**
**7214 W. 71st Terrace**
**Overland Park Kansas 66204 (US)**
Inventor: **Rutter, Jerry Lynn**
**6128 Norwood**
**Mentor Ohio 44060 (US)**

(74) Representative: **Huskisson, Frank Mackie et al,**
**FITZPATRICKS 4 West Regent Street**
**Glasgow G2 1RS Scotland (GB)**

**EP 0 019 369 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Isothioureido isoindolediones, process for their preparation and use as plant regulators

Description of the Invention

Growth regulating effects have been observed upon application of many chemical substances to plants. In general, very few of these substances can be used with benefit to the plants which are affected. In most instances the beneficial effects, if any, are minor and the major effects are so drastic that the compounds can only be used for the destruction of the plants. Examples of compounds with drastic effects which have become useful as herbicides are 2,4—D, EPTC and alachlor. Although a relatively small number of growth regulants has proved to be commercially useful, there are many uses for active compounds, among which are the following:

Increase or induce flowering (pineapple).

Increase blossom set, pod set, seed set, and/or fruit set (prevent abortion of flowers or withered blossoms).

Increase size of fruits, vegetables, seed, and/or tubers (grapes, soybeans, sugar beets, etc.)

Decrease size of fruits, vegetables, seed, and/or tubers (potatoes, and grapefruits).

Increase number of tillers (cereals).

Increase number of shoots from crown (alfalfa).

Increase branching (soybeans) or widen branches (apples).

We have now discovered a group of novel compounds which display a great variety of growth regulating effects, indicating utility for many purposes, including uses mentioned above.

Briefly, the novel class of growth regulant compounds has the general structural formula:

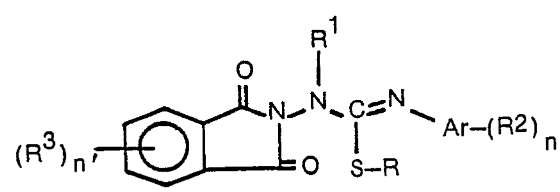

In which R is $C_1$ to $C_4$ alkyl, alkenyl or alkynyl to which may be attached phenyl, pyridyl, benzoyl, phenoxy, halophenoxy, halobenzoyl, N-phenylcarbamyl, N-ethyl-N-phenylcarbamyl, N-trifluoromethylthiadiazolylcarbamyl, ethoxycarbonyl, t-butylcarbonyl, methylthio, phenylcarbonyl, 4-Br-phenyl, 2,6-di-Cl phenyl, or halo substituents,

$R^1$ is H, $C_1$ to $C_3$ alkyl, alkenyl or alkynyl to which may be attached phenyl, halophenyl or phenoxy groups, acetyl ethoxycarbonyl, or R and $R^1$ together may be 1,4-butadienyl,

Ar is phenyl or benzoyl,

$R^2$ is $C_1$ to $C_4$ alkyl, alkoxy, diethylamino, phenoxy, benzyloxy, carbalkoxy, acetyl, methylenedioxy, trifluoromethyl, nitro, halo or cyano and n may be zero or an integer from 1 to 4, and when $n = 2$ $R^2$ may be 3,4-propylene, with the provision that at least one position ortho to the point of attachment of a phenyl ring of the Ar structure must be unsubstituted,

$R^3$ is $C_1$—$C_4$ alkyl or halo and n' may be zero or an integer from 1 to 4.

By virtue of their basic chemical characteristics, the novel compounds may also exist in the form of salts, by addition of a strong acid, such as HBr or HCl. In the salt form the compounds are more easily formulated in water-soluble and water-dispersible formulations.

By selection of the substituent R, the lipophilic properties of the novel compounds may be modified to obtain desirable improvements in ease of formulation, particularly as emulsifiable concentrates, and also improvements in activity on many plant species.

The nature of the substituent group $R^1$ is important to the intensity of the growth regulator effect. For highest activity, small alkyl or alkenyl groups having one to three carbon atoms are preferred.

The nature of the $R^2$ substituents is not as critical as the aforementioned groups. However, both activity and selectivity of action may be adjusted to some degree by selection of these groups. In general, $C_1$ to $C_4$ alkyl or alkoxy, halo or cyano substituents are preferred and Ar is preferably phenyl.

In general the substituents designated $R^3$ do not enhance appreciably the activity of the growth regulators. However, by choice of type and position of the $R^3$ substituents, chemical stability may be improved and the growth regulator effect may be prolonged. Prolonging the effect may in some instances eliminate the need for a second application, so that the compounds may be utilized more efficiently. Unless this is economically advantageous, however, compounds in which $n' = 0$ are preferred.

## Synthesis of the Growth Regulants

The novel compounds of this invention may be manufactured by means of the general method comprising reacting a compound of the formula:

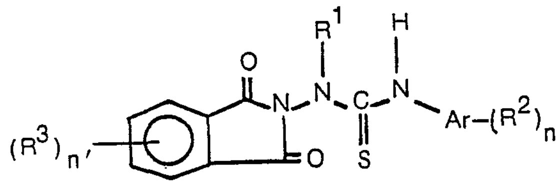

in a non-reactive polar organic solvent with a compound of the formula R—X in which X is a leaving group exemplified by a chlorine, bromine, iodine or sulfate substituent. Specific procedures which are suitable for synthesis of representative compounds are outlined in the following chart and exemplified below.

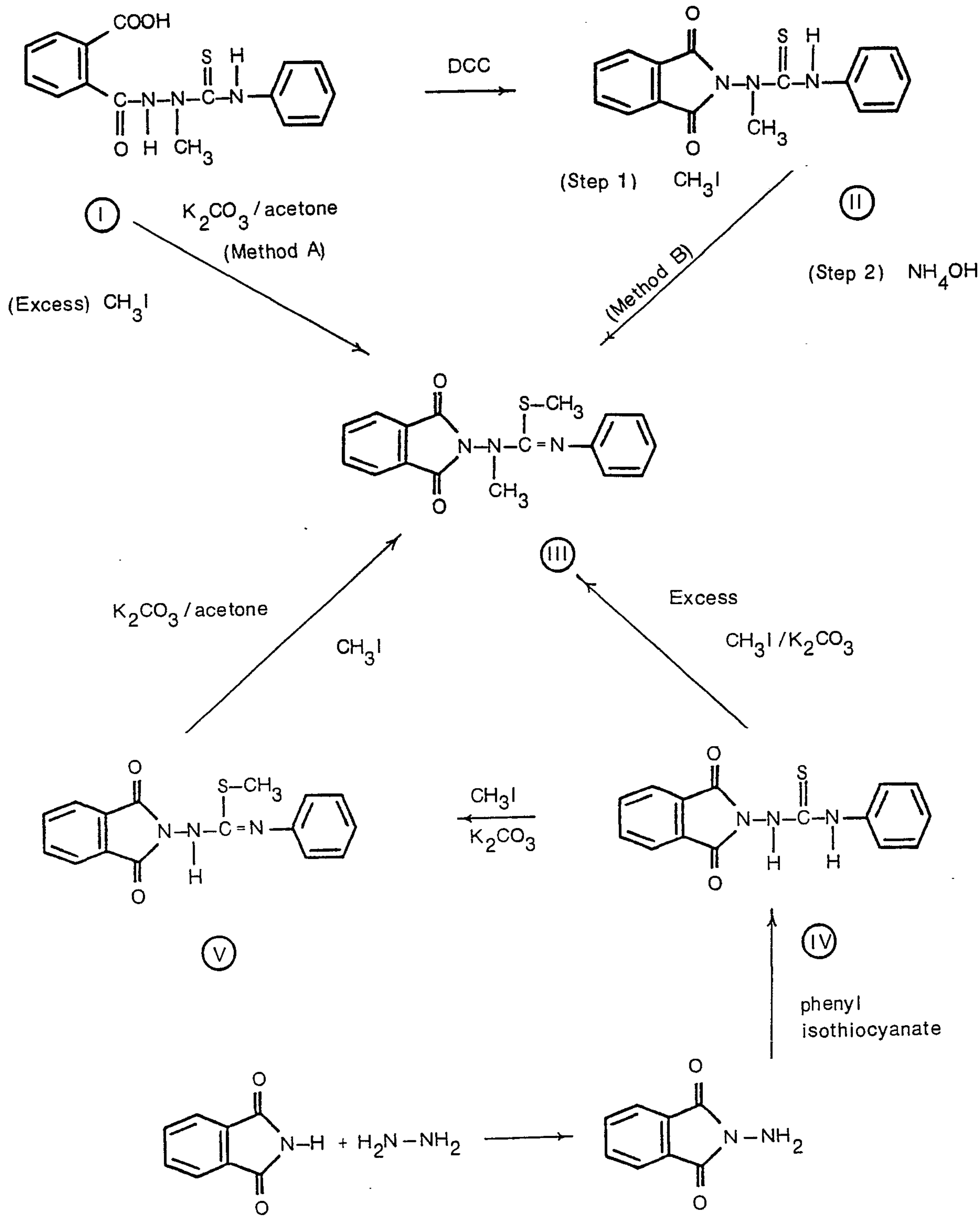

It will be understood that when it is desired that R and $R^1$ shall be different, the alkylation at the sulfur and amino locations is conveniently done in separate reaction steps. The following specific procedures illustrate the general methods of synthesis.

Synthesis of methyl hydrogen phthalate

Methanol (300 ml) was added in a single portion to 148 g (1.00 mole) of phthalic anhydride and the resulting suspension was stirred and heated at reflux for 36 hours; solution occurred during heating. The solvent was removed and the product was recrystallized from a mixture of ethyl acetate and hexane to afford 114.6 g of the title compound (Lit.: Beilstein, *9*,797 — mp 82.5°, 84°).

Synthesis of methyl phthaloyl chloride

Methyl hydrogen phthalate (110.0 g., 0.611 mole) and thionyl chloride (77.4 g., 0.650 mole) were mixed in 200 ml of chloroform, keeping the temperature below 30°. After stirring for two hours at room temperature, the system was heated at reflux for five hours. The solvent was evaporated at reduced pressure and the crude product (120.9 g) was used without further purification (Lit.: Beilstein, *9*, 797 — no constants).

Synthesis of 2-(2-carboxybenzoyl)-1-methyl-N-phenylhydrazine thiocarboxamide (I)

This compound was made by reacting phthalic anhydride with 1-methyl-N-phenylhydrazinethiocarboxamide according to conventional procedures, as follows:

The hydrazinethiocarboxamide was dissolved in about 75 ml of dimethylformamide and placed in a 3-necked round-bottomed flask equipped with magnetic stirrer, condenser, additional powder funnel and thermometer. The anhydride was added in portions at 20°. The contents were stirred overnight at room temperature, then poured into ice water the following morning. The resulting solid was recrystallized in hexane and ethanol (m.p. 155—156°C.)

Optionally ring substituted phthalic anhydrides and substituted 2-carbomethoxybenzoyl chlorides may be condensed with substituted 1-methylhydrazinethiocarboxamides to give desired intermediate compounds as in the following scheme:

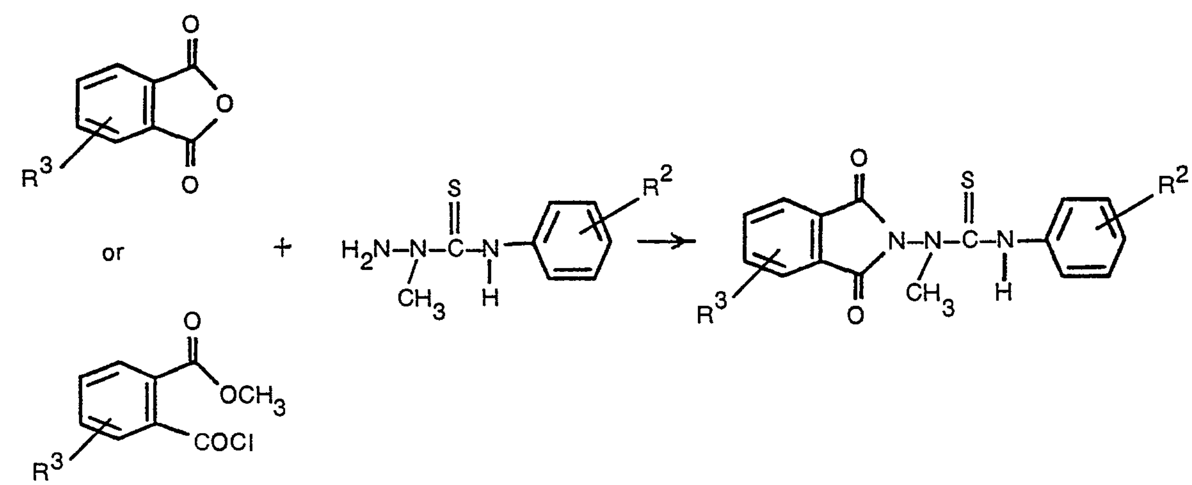
or

These methods are illustrated in the following specific procedures. The identity of the product was confirmed in each instance by means of infrared and nuclear magnetic resonance spectra. All melting points are as determined, uncorrected (Degrees C.).

N-Methyl-N-(phenylthiocarbamoyl)-2-amino-1H-isoindole-1,3-(2H)-dione (II)

To an ice-cold solution of 8.25 g (0.025 mole) of 2-(2-carboxybenzoyl)-1-methyl-N-phenylhydrazinethiocarboxamide in 225 ml of 1,2-dimethoxyethane at ~2°C, a solution of 5.5 g (0.027 mole) of N,N′-dicyclohexylcarbodiimide was added dropwise below 5°C with stirring. The mixture was stirred in the ice bath and then left at room temperature overnight. The mixture was filtered to remove N,N′-dicyclohexylurea and the filtrate was evaporated below 40°C, under vacuum, to give a yellow amorphous solid which was stirred in 100 ml of dry ether and warmed gently. The ether solution was allowed to stand for a few hours and filtered to give 4.6 g (59%) of whitish yellow crystals, m.p. 142—144°.

Recrystallization from ethyl acetate-hexane gave whitish crystals, m.p. 151—153°.

Mass spectrum: $M^+$ 311

N-Methyl-N-(phenylthiocarbamoyl)-2-amino-4-methyl-1H-isoindole-1,3-(2H)dione

To a solution of 6.8 g (0.037 mole) of 1-methyl-N-phenylhydrazinethiocarboxamide and 3.0 g of

pyridine in 100 ml dry dimethoxyethane, 2-carbomethoxy-6-methylbenzoyl chloride (8.0 g, 0.037 mole) was added and the resulting mixture was stirred at room temperature for 60 hours. The solvent was distilled and the residue was taken up in ethyl acetate, filtered and dried on anhydrous magnesium sulfate. Removal of the solvent gave 10 g (83%) of the desired product, m.p. 110—115° (dec.).

2-Amino-1H-isoindole-1,3-(2H)dione

To an ice-cold suspension of 14.7 g (0.1 mole) of phthalimide in 100 ml of 95% ethyl alcohol at 5°C, with stirring, 3.6 ml (0.11 mole) of 96.8% hydrazine was added dropwise. A slight exothermic reaction was observed and the mixture was allowed to stir at 5°C for two hours. The mixture was diluted with 200 ml of ice water, stirred, filtered, washed with water and dried to give 12.2 g (75%) of white powder, m.p. 199—202°.

Recrystallization from methanol-water gave white needles, m.p. 201—203°.

N-(Phenylthiocarbamoyl)-2-amino-1H-isoindole-1,3-(2H)dione (IV)

To a suspension of 8.2 g (0.05 mole) of 2-amino-1H-isoindole-1,3-(2H)dione in 50 ml of dry 2-propanol, 6 ml (0.05 mole) of phenyl isothiocyanate was added. The mixture was stirred and refluxed for 3 hours, allowed to cool to room temperature and poured into 300 ml of 50% ethyl alcohol. After stirring for one hour, the solid which formed was filtered, washed with water and dried to give 12.1 g (81%) of the desired product as a white powder, m.p. 180—181°. To make compounds in which Ar is benzoyl, benzoyl isothiocyanates may be employed in procedures of the type described above.

Example 1

Preparation of 2-(1,2-Dimethyl-3-phenylisothioureido)-1H-isoindole-1,3-(2H)-dione (III)

*Method A*

To a slurry of 3.3 g (0.01 m) of 2-(2-carboxybenzoyl)-1-methyl-N-phenylhydrazinethiocarboxamide and 4.1 g (0.03 m) of potassium carbonate in 100 ml of acetone, was added 4.3 g (0.03 m) of methyl iodide. After stirring at room temperature for 16 hrs, 500 ml of ice water was added and the pH adjusted to 3 with dil HCl. An oil separated which crystallized with continued stirring, was collected and washed with water to give 1.2 g (37% yield) of product, m.p. 88—90°.

*Method B*

Six grams (0.019 m) of N-Methyl-N-(phenylthiocarbamoyl)-2-amino-1H-isoindole-1,3-2H-dione was dissolved in 2 ml of DMF and 5 ml of methyl iodide, then stirred at room temperature for 16 hours. To the solid mass was added ethyl acetate and the crystals collected, washed with ethyl acetate and dried to give 7.3 g of hydroiodide salt, m.p. 145—148° (dec).

The free base was obtained by suspending the salt in dilute $NH_4OH$ and extracting with $CHCl_3$ which was washed with water, then with saturated aqueous NaCl and dried over anhydrous $Na_2SO_4$. Removal of the $CHCl_3$ at reduced pressure in a rotary evaporator gave a residual oil which was crystallized from ether-petroleum ether, wt. 4.6 g, m.p. 90—91° (75%).

Example 2

Preparation of 2-(2-Methyl-3-phenylisothioureido)-1H-isoindole-1,3-(2H)dione (V)

To 100 ml of dry acetone, 5.0 g (0.018 m) of N-(phenylthiocarbamoyl)-2-amino-1-H-isoindole-1,3-(2H)dione and 2.5 g (0.018 m) powdered anhydrous potassium carbonate was added. To this suspension 1.1 ml (0.018 m) of methyl iodide was added and the mixture was stirred overnight at room temperature. The mixture was poured into ~300 ml of water, stirred, filtered, washed with water and dried to give 4.4 g (78%) of a yellow powder, m.p. 161—164°. Mass spectrum: M+311.

Example 3

Preparation of 2-(1-Allyl-2-methyl-3-phenylisothioureido)-1-H-isoindole-1,3-(2H)dione

To 50 ml of dry acetone, 4.0 g (0.013 m) of the product of Example 2, 2.2 g (0.016 m) of powdered anhydrous potassium carbonate, and 1.4 ml (0.016 m) of allyl bromide was added in succession and the mixture stirred overnight at room temperature. The mixture was poured into 300 ml of ice water with stirring. The aqueous layer was decanted and the residue suspended in fresh ice cold water, stirred and filtered to give a yellow semisolid product. This was purified by stirring in hexane to give a yellow powder 1.8 g (39%), m.p. 78—81°.

Example 4

Preparation of 2-(1,2-Diallyl-3-phenylisothioureido)-1-H-isoindole-1,3-(2H)dione

A mixture of 100 ml dry acetone, 5.0 g (0.018 m) N-(phenylthiocarbamoyl)-2-amino-1-H-isoindole-1,3-(2H)dione, 6.9 g (0.05 m) powdered anhydrous potassium carbonate, and 4.3 ml (0.05 m) allyl bromide was stirred overnight at room temperature and evaporated to dryness. The residue was suspended in 50 ml of cold water and the product was extracted with ether. The ether extract, on work-up, gave 4.7 g (69%) of thick yellow liquid.

Use of the Growth Regulators

The growth of plants may be regulated by applying the growth regulator compounds to the plants, either on seed, the soil or directly on the plants in an effective amount. If the objective is to combat unwanted vegetation, an amount sufficient to produce severe injury or abnormality of form of growth is usually sufficient. Sometimes the unwanted vegetation continues to live for a time but is stunted or distorted so that it cannot compete with crop plants and eventually succumbs to shading by the crop, or failure to obtain or to assimilate nutrients in the manner of a normal plant. An effect which is occasionally observed upon pre-emergent application is a loss of geotropic orientation upon germination of seed. In extreme cases roots may grow upward out of the soil, while leaves remain beneath the surface. Naturally the plants do not survive for long in this upside-down orientation. However, they can be transplanted and made to survive, which indicates that the growth regulant in one sense is not very phytotoxic. The survival rate of affected plants is much greater when the growth regulants are applied post-emergently, because the plants are already past the critical stages of emergence and growth of foliage and a root system. Retarded growth, with some abnormalities of form, accompanied by an increase in chlorophyl concentration in foliage is sometimes observed after post-emergent application. This combination of properties is particularly desirable for use on turf, to obtain a good color and reduce the frequency of cutting.

In highly active compounds, phytotoxic effects of pre-emergent and post-emergent application are often readily apparent. These effects may be demonstrated by means of the following illustrative procedures.

Pre-emergent Application

Disposable paper trays about 2 1/2 inches deep were filled with soil and sprayed with aqueous spray mixtures at a rate of 5 lb. of active chemical per acre of sprayed area, were seeded with 6 species of plant seeds and were then covered with about 1/4 inch of soil. The spray mixtures were prepared by dissolving the proper amount of growth regulant compound in 15 ml of acetone, adding 4 ml of a solvent-emulsifier mixture consisting of 60 wt. percent of a commercial polyoxyethylated vegetable oil emulsifier (96 wt. percent active ingredient, Emulphor EL—719), 20 wt. percent xylene and 20 wt. percent deodorized kerosene, then bringing total volume up to 60 ml by addition of warm water. Twenty-one days after seeding and treatment the plantings were examined and plant injury was rated according to the following schedule.

DEGREE OF EFFECT

0 = no effect
1 = slight effect, plants recovered
2 = moderate effect, injury to 26 to 75 percent
3 = severe effect, injury to 76 to 99 percent of foliage
4 = maximum effect (all plants died)

Post-emergent Application

Several species of plants were grown in potting soil in disposable styrofoam trays and tomatoes were grown in four-inch pots in the greenhouse. Aqueous spray formulations were prepared and the growing plants were sprayed at a spray volume of 60 gallons per acre and an application rate of 5 lb. per acre. Spray mixtures were prepared in the manner described above. For comparative purposes, plants were also sprayed at 60 gal./acre with a spray mixture containing no growth regulator. Plant injury was again rated according to the schedule disclosed above and observations of growth regulant effects in both pre- and post-emergent tests were observed and recorded as follows:

| Effect | Abbreviation in Tables. |
|---|---|
| Formative effect on new growth | F |
| Epinasty | E |
| Growth Reduction | G |
| Necrosis | N |
| Non-emergence | K |

In the table below there are tabulated various compounds which have been made according to the above illustrative procedures, as well as observations of pre- and post-emergent herbicidal and growth regulant effects. Some of the compounds were tested in the form of the hydro-halide salts, resulting from alkylation with alkyl halides. These compounds are identified under the column titled "salt type".

TABLE I
Part A
Compounds of the formula

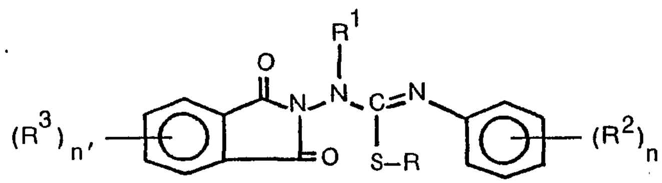

| Compnd. No. | R | $R^1$ | $(R^2)n$ | $(R^3)n'$ | Salt Type | M.P. °C. | Comments on Utility |
|---|---|---|---|---|---|---|---|
| 2878 | $—CH_3$ | $—CH_3$ | n = 0 | n′ = 0 | | 88—90° | Increases chlorophyl in foliage |
| 2960 | allyl | $—CH_3$ | n = 0 | n′ = 0 | | oil | Increases chlorophyl in foliage. Epinesty on alfalfa. |
| 2961 | benzyl | $—CH_3$ | n = 0 | n′ = 0 | | oil | Increases pod set of soybeans. |
| 3062 | $—CH_3$ | —H | n = 0 | n′ = 0 | | 161—4° | Defoliates cotton. Kills pigweed. |
| 3099 | $—CH_3$ | $—CH_3$ | 3-F | n′ = 0 | | 62—5° | Defoliates cotton. Kills pigweed. |
| 3100 | $—CH_2CH_3$ | $—CH_3$ | n = 0 | n′ = 0 | | 80—2° | Defoliates cotton. Kills pigweed. |
| 3101 | allyl | $—CH_3$ | 3-F | n′ = 0 | | oil | |
| 3102 | benzyl | $—CH_3$ | 3-F | n′ = 0 | | oil | |
| 3104 | $—CH_3$ | $—CH_3$ | $2,4-(CH_3)_2$ | n′ = 0 | | 107—9° | |
| 3105 | n-propyl | $—CH_3$ | n = 0 | n′ = 0 | | oil | Defoliates cotton. Kills pigweed. |
| 3110 | 4-Br-Benzyl | $—CH_3$ | n = 0 | n′ = 0 | | oil | Controls pigweed. |

TABLE I
Part A (continued)

Compounds of the formula

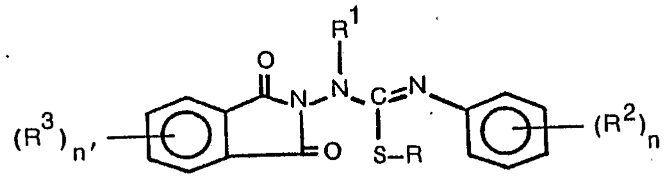

| Compnd. No. | R | $R^1$ | $(R^2)n$ | $(R^3)n'$ | Salt Type | M.P. °C | Comments on Utility |
|---|---|---|---|---|---|---|---|
| 3111 | $-CH_2CO-C(CH_3)_3$ | $-CH_3$ | n = 0 | n' = 0 | HBr | 135—8° | Promotes tillering of oats. |
| 3129 | $-CH_3$ | $-CH_3$ | 2,6-$(CH_3)_2$ | n' = 0 | | 137—40° | |
| 3130 | $-CH_3$ | $-CH_3$ | 3,4- $-CH=CH-CH=CH-$ | n' = 0 | | wax | Kills lambsquarter (post-emerg.) |
| 3134 | $-CH_3$ | $-CH_3$ | 2,4,5-$(CH_3)_3$ | n' = 0 | | oil | |
| 3135 | $-CH_3$ | $-CH_3$ | 2,5-$(CH_3)_2$ | n' = 0 | | 122—4° | |
| 3141 | $-CH_3$ | $-CH_3$ | n = 0 | n' = 0 | HI | 145—8° dec. | Defoliates cotton. Kills pigweed. |
| 3143 | Allyl | $-CH_3$ | n = 0 | n' = 0 | HBr | 117—8° | Controls pigweed. |
| 3144 | benzyl | $-CH_3$ | n = 0 | n' = 0 | HBr | 147—8° | Defoliates cotton. Kills pigweed. |
| 3171 | $-CH_2-CH_2-$ | | n = 0 | n' = 0 | | 75—80° | |
| 3176 | $-CH_3$ | Allyl | n = 0 | n' = 0 | | 78—81° | Promotes tillering of oats |
| 3191 | $-CH_3$ | $-CH_3$ | —2,3-$(CH_3)_2$ | n' = 0 | | 95—7° | |
| 3192 | $-CH_3$ | $-CH_3$ | 4-phenoxy | n' = 0 | | 159—62° | |
| 3193 | $-CH_3$ | $-CH_3$ | 4-n-butyl | n' = 0 | | oil | |

TABLE I
Part A (continued)
Compounds of the formula

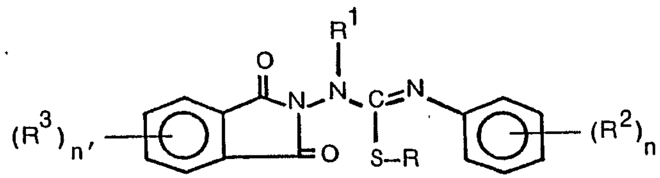

| Compnd. No. | R | $R^1$ | $(R^2)n$ | $(R^3)n'$ | Salt Type | M.P. °C. | Comments on Utility |
|---|---|---|---|---|---|---|---|
| 3205 | 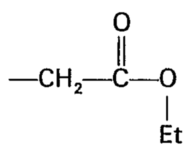 | $—CH_3$ | n = 0 | n′ = 0 | HBr | 166—8° | |
| 3206 | ″ | $—CH_3$ | n = 0 | n′ = 0 | | 67—70° | |
| 3215 | $—CH_3$ | $CH_2C{\equiv}CH$ | n = 0 | n′ = 0 | | oil | |
| 3216 | allyl | allyl | n = 0 | n′ = 0 | | oil | Increases tomato fruit set. (Heart shaped fruit) |
| 3218 | $—CH_3$ | —H | $4\text{-}CH_3$ | n′ = 0 | | 151—3° | |
| 3219 | allyl | —H | $4\text{-}CH_3$ | n′ = 0 | | 128—30° | |
| 3220 | $—CH_3$ | $—CH_3$ | $4\text{-}CH_3$ | n′ = 0 | | 111—114° | |
| 3221 | $—CH_3$ | allyl | $4\text{-}CH_3$ | n′ = 0 | | oil | |
| 3222 | allyl | —H | n = 0 | n′ = 0 | | 60—61° | Increases tomato fruit set. |
| 3251 | 3,3-dimethyl allyl | —H | n = 0 | n′ = 0 | | 115—7° | |

TABLE I
Part A (continued)
Compounds of the formula

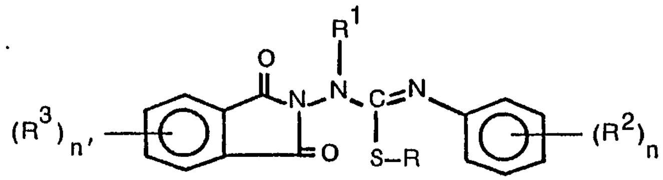

| Compnd. No. | R | R¹ | (R²)n | (R³)n′ | Salt Type | M.P. °C. | Comments on Utility |
|---|---|---|---|---|---|---|---|
| 3274 | $—CH_3$ | 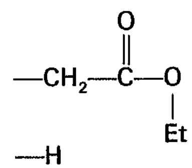 | n = 0 | n′ = 0 | | oil | |
| 3276 | $—CH_3$ | —H | $—3—CH_3$ | n′ = 0 | | 158—9° | |
| 3277 | $—CH_3$ | $—CH_3$ | $—3—CH_3$ | n′ = 0 | | oil | Increases tomato fruit set, tillering of oats. |
| 3278 | allyl | allyl | $—3—CH_3$ | n′ = 0 | | oil | |
| 3292 | $—CH_2C{\equiv}CH$ | $—CH_3$ | —3—F | n′ = 0 | HBr | 140—1° | Increases fruit set on tomatoes, tillering of oats, controls crabgrass. |
| 3293 | $—CH_2C{\equiv}CH$ | $—CH_3$ | —3—F | n′ = 0 | | 90—2° | |
| 3294 | $—CH_3$ | $—CH_3$ | —3—Cl | n′ = 0 | | oil | Increases tomato fruit set, tillering of oats, controls crabgrass. |
| 3295 | $—CH_3$ | $—CH_3$ | $—3,4-Cl_2$ | n′ = 0 | | 124—7° | Increases tillering of oats. |
| 3296 | $—CH_3$ | —H | —3-Cl | n′ = 0 | | 198—200° | |
| 3297 | $—CH_3$ | —H | $—3,4-Cl_2$ | n′ = 0 | | 182—3° | |
| 3298 | $—CH_3$ | allyl | 3-Cl | n′ = 0 | | 102—4° | |

TABLE I
Part A (continued)
Compounds of the formula

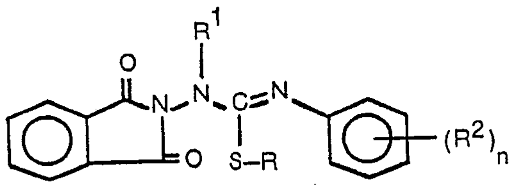

| Compnd. No. | R | $R^1$ | $(R^2)n$ | Salt Type | M.P.°C | Comments on Utility |
|---|---|---|---|---|---|---|
| 3283 | allyl | $—CH_3$ | 2,5-$(CH_3)_2$ | HBr | 129—130 | |
| 3287 | allyl | $—CH_3$ | 2,5-$(CH_3)_2$ | | Syrup | |
| 3409 | $—CH_2COOC_2H_5$ | " | 4-F | HBr | 169—171 | |
| 3410 | $—CH_2COOC_2H_5$ | " | 4-F | | 83—85 | |
| 3411 | $—CH_2COOC_2H_5$ | " | 3-F | HBr | 138—140 | |
| 3497 | $—C_2H_5$ | —H | n = 0 | | 130—138 | |
| 3500 | $—C_2H_5$ | allyl | n = 0 | | Syrup | |
| 3627 | $—CH_3$ | $—CH_3$ | 3-$CF_3$ | | oil | Increases fruit set |
| 3628 | $—CH_3$ | $—CH_3$ | 4-Cl | | 123—125 | Increases fruit set |
| 3629 | $—CH_2CH_2CH_2—$ | | 4-Cl | | 100—130 | |
| 3631 | benzyl | —H | 4-Cl | | 168—170 | |
| 3632 | benzyl | $—CH_3$ | 4-Cl | | 113—115 | Increases fruit set |
| 3633 | benzyl | 2-propynyl | 4-Cl | | oil | Increases fruit set |
| 3635 | benzyl | benzyl | 4-Cl | | 103—105 | Increases fruit set |
| 3637 | $—CH_3$ | acetyl | 3,4-$Cl_2$ | | 143—145 | |

TABLE I
Part A (continued)
Compounds of the formula

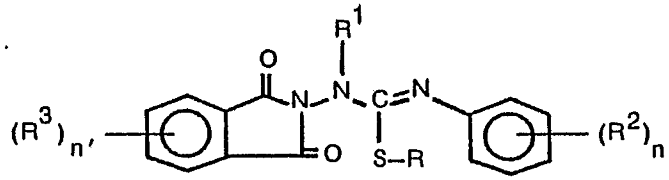

| Compnd. No. | R | $R^1$ | $(R^2)n$ | $(R^3)n'$ | Salt Type | M.P. °C. | Comments on Utility |
|---|---|---|---|---|---|---|---|
| 3299 | $—CH_3$ | $—CH_2C{\equiv}CH$ | 3-Cl | $n' = 0$ | | oil | |
| 3300 | $—CH_3$ | 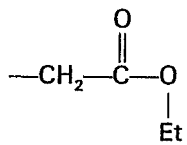 | 3-Cl | $n' = 0$ | | oil | |
| 3302 | $—CH_3$ | allyl | $3,4-Cl_2$ | $n' = 0$ | | 90—2 | |
| 3303 | $—CH_3$ | $—CH_2C{\equiv}CH$ | $3,4-Cl_2$ | $n' = 0$ | | oil | |
| 3339 | $—CH_3$ | benzyl | $3,4-Cl_2$ | $n' = 0$ | | oil | |
| 3348 | $—CH_2CH_2CH_2—$ | | $n = 0$ | $n' = 0$ | | 193—5 | |
| 3340 | n-butyl | —H | $—3,4-Cl_2$ | $n' = 0$ | | 165—7 | |
| 3342 | $—CH_2—S—CH_3$ | —H | —3-Cl | $n' = 0$ | | crude oil | |
| 3344 | n-butyl | $—CH_3$ | $3,4-Cl_2$ | $n' = 0$ | | 98—101 | Increased fruit set |
| 3346 | $—CH_2—S—CH_3$ | $—CH_3$ | 3-Cl | $n' = 0$ | | 77—80 | Increased fruit set |
| 3328 | $—CH_3$ | —H | $n = 0$ | $—3-CH_3$ | | 180—4 | |
| 3329 | $—CH_3$ | $—CH_3$ | $n = 0$ | $—3-CH_3$ | | oil | |
| 3331 | $—CH_3$ | $—CH_3$ | $n = 0$ | $—3-CH_3$ | | 135—136 | |
| 3332 | $—CH_3$ | —H | $n = 0$ | 4,5,6,7-tetrachloro | | 265—70 | |

TABLE I
Part A (continued)
Compounds of the. formula

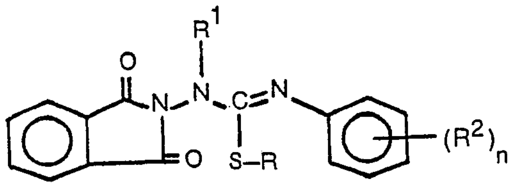

| Compnd. No. | R | $R^1$ | $(R^2)n$ | Salt Type | M.P.°C. | Comments on Utility |
|---|---|---|---|---|---|---|
| 3700 | $—CH_3$ | $—CH_3$ | $4\text{-}NO_2$ | | 152—155 | Oat tillered increased fruit set |
| 3701 | benzyl | —H | $4\text{-}NO_2$ | | 182—184 | |
| 3702 | $—CH_2COC_6H_5$ | " | —H | | 149—152 | |
| 3703 | 4-pentenyl | " | —H | | 79—81 | |
| 3704 | 2,6-dichloro-benzyl | —H | —H | | 176—178 | All plants taller except tomato |
| 3705 | 4-methyl-benzyl | —H | —H | | 163—165 | |
| 3706 | 3-phenoxy-propyl | —H | —H | | 100—103 | Alfalfa very tall |
| 3707 | benzyl | $—CH_3$ | $4\text{-}NO_2$ | | 122—125 | Increased fruit set |
| 3708 | benzyl | 2-propynyl | $4\text{-}NO_2$ | | 94—97 | |
| 3709 | 2,6-dichloro-benzyl | $—CH_3$ | —H | | 165—167 | Increased fruit set |
| 3710 | 4-methyl-benzyl | $—CH_3$ | —H | | Oil | Increased fruit set and oat tillers |
| 3731 | $—CH_3$ | —H | $4\text{-}OCH_3$ | | 197—199 | Increased fruit set |
| 3732 | $—C_2H_5$ | —H | $4\text{-}OCH_3$ | | 195—197 | |
| 3733 | $—CH_3$ | $—CH_3$ | $3\text{-}Cl_1$ $4\text{-}CH_3$ | | 126—129 | Increases fruit set |
| 3734 | $—C_2H_5$ | —H | $3\text{-}Cl_1$ $4\text{-}CH_3$ | | 166—168 | |
| 3749 | propyl | —H | —H | | 104—106 | |
| 3750 | propyl | allyl | —H | | 80—82 | Increased oat tillered fruit set |
| 3751 | isopropyl | —H | —H | | 132—134 | |
| 3752 | isopropyl | $—CH_3$ | —H | | dec. 85 | Increased fruit set |

TABLE I
Part A (continued)

Compounds of the formula

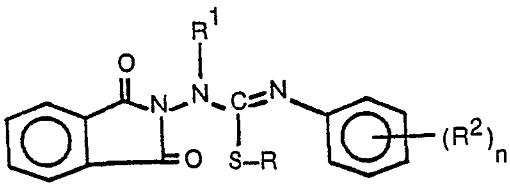

| Compnd. No. | R | $R^1$ | $(R^2)n$ | Salt Type | M.P.°C | Comments on Utility |
|---|---|---|---|---|---|---|
| 3780 | $-CH_3$ | $-CH_3$ | 4-$OCH_3$ | | 105—108 | |
| 3781 | $-C_2H_5$ | $-CH_3$ | 4-$OCH_3$ | | 85—88 | |
| 3782 | $-C_2H_5$ | $-CH_3$ | 3Cl-<br>4-$CH_3$ | | 104—106 | Increased fruit set |
| 3783 | $-CH_3$ | $-CH_3$ | 2-$CH_3$ | | Oil | Increased fruit set |
| 3784 | $-C_2H_5$ | —H | 2-$CH_3$ | | 135—137 | |
| 3785 | $-C_2H_5$ | $-CH_3$ | 2-$CH_3$ | | Oil | Increases fruit set |
| 3786 | $-CH_3$ | $-CH_3$ | 2-Cl | | Oil | Increases fruit set |
| 3787 | $-C_2H_5$ | —H | 2-Cl | | 116—118 | |
| 3788 | $-CH_3$ | $-CH_3$ | 4-F | | 82—85 | Increases fruit set |
| 3789 | $-C_2H_5$ | —H | 4-F | | 183—185 | |
| 3790 | $-CH_3$ | —H | 3-F | | 204—206 | Increases fruit set |
| 3791 | $-C_2H_5$ | $-CH_3$ | 2-Cl | | Oil | Increases fruit set |
| 3792 | $-C_2H_5$ | $-CH_3$ | 4-F | | Oil | Increases fruit set |
| 3793 | $-CH_3$ | —H | 2-$CH_3$<br>3-Cl | | 165—168 | |
| 3794 | $-CH_3$ | —H | 4-butyl | | 127—130 | |
| 3795 | $-C_2H_5$ | —H | 4-butyl | | 107—109 | |
| 3796 | $-CH_3$ | $-CH_3$ | 4-isopropyl | | 111—114 | Increases fruit set |

TABLE I
Part A (continued)

Compounds of the formula

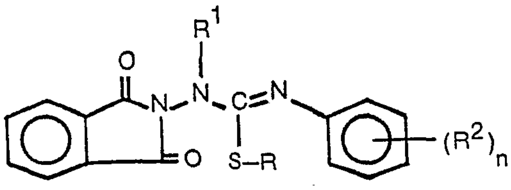

| Compnd. No. | R | $R^1$ | $(R^2)n$ | Salt Type | M.P.°C. | Comments on Utility |
|---|---|---|---|---|---|---|
| 3797 | $-C_2H_5$ | —H | 4-$C_3H_7$ | | 142—144 | |
| 3798 | $-CH_3$ | $-CH_3$ | 2-$Cl_1$<br>6-$CH_3$ | | 106—108 | |
| 3799 | $-C_2H_5$ | —H | 2-$Cl_1$<br>6-$CH_3$ | | 172—174 | |
| 3800 | benzyl | —H | —H | | 115—116 | |
| 3812 | isopropyl | allyl | —H | | 87—90 | Increased tillers, fruit set |
| 3813 | butyl | —H | —H | | 119—121 | |
| 3814 | butyl | $-CH_3$ | —H | | dec.65 | Increased tillers, fruit set |
| 3815 | butyl | allyl | —H | | Syrup | Increased fruit set |
| 3816 | isobutyl | —H | —H | | 137—139 | |
| 3849 | isobutyl | $-CH_3$ | —H | | dec.65 | Increased fruit set, tillers |
| 3850 | isobutyl | allyl | —H | | 62—65 | Increased fruit set |
| 3851 | $-CH_2\overset{\overset{O}{\|}}{C}OC_2H_5$ | —H | —H | | semi-solid | |
| 3977 | —CN | $-CH_3$ | n = 0 | | dec.145 | |
| 4017 | $-CH_3$ | —H | 3-benzyloxy | | 106—108 | |
| 4019 | $-CH_3$ | —H | 3-$NO_2$ | | 191—193 | Increases fruit set |
| 4020 | $-C_2H_5$ | —H | 3-$NO_2$ | | 192—195 | Increases fruit set |
| 4021 | $-CH_3$ | —H | 4-benzyloxy | | 130—133 | |
| 4023 | $-CH_3$ | $-CH_3$ | 3CF3—<br>4-Cl | | 95—97 | Increases fruit set, tillers |
| 4025 | $-CH_3$ | —H | 2-$CF_3$ | | 156—159 | Increases fruit set, tillers |

TABLE I
Part A (continued)

Compounds of the formula

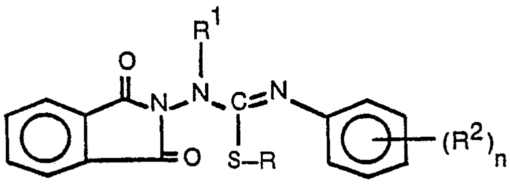

| Compnd. No. | R | $R^1$ | $(R^2)n$ | Salt Type | M.P.°C. | Comments on Utility |
|---|---|---|---|---|---|---|
| 4026 | $-CH_3$ | $-CH_3$ | 3-benzyloxy | | 83—85 | |
| 4027 | $-CH_3$ | $-CH_3$ | 3-$NO_2$ | | 105—108 | Increased fruit set, elongated growth |
| 4028 | $-CH_3$ | $-CH_3$ | 4-benzyloxy | | 87—90 | |
| 4029 | $-CH_3$ | $-CH_3$ | 2,6-$Cl_2$ | | Oil | |
| 4030 | $-CH_3$ | $-CH_3$ | 2-$CF_3$ | | Oil | Increased fruit set, tillers |
| 4033 | $-CH_3$ | —H | 3-$OCH_3$ | | 158—160 | Increased fruit set |
| 4034 | $-C_2H_5$ | —H | 3-$OCH_3$ | | 103—105 | |
| 4035 | $-CH_3$ | —H | 3,5-$(CH_3)_2$ | | 200—202 | Increased fruit set |
| 4036 | $-C_2H_5$ | —H | 3,5-$(CH_3)_2$ | | 129—131 | |
| 4062 | 2-(2,4-dichloro-phenoxy) ethyl | $-CH_3$ | n = 0 | | 108—110 | Increased fruit set, elongated growth |
| 4063 | $-CH_3$ | $-CH_3$ | 3-$OCH_3$ | | 85—88 | Increased fruit set and tillers |
| 4064 | $-CH_3$ | $-CH_3$ | 3-$SCH_3$ | | 91—94 | Fruit set, tillers |
| 4065 | $-C_2H_5$ | —H | 3-$SCH_3$ | | 127—129 | |
| 4066 | $-CH_3$ | $-CH_3$ | 2-$Cl_1$ 4-$CH_3$ | | 90—92 | More fruit set |
| 4068 | $-CH_3$ | $-CH_3$ | 3-$C(=O)CH_3$ | | 109—111 | Fruit set tillers |
| 4070 | $-CH_3$ | $-CH_3$ | 4-$COCH_3$ | | 144—145 | Fruit set |
| 4115 | $-CH_3$ | $-CH_3$ | 4-CN | | 175—178 | Fruit set |
| 4116 | $-CH_3$ | —H | 4-$N(C_2H_5)_2$ | | 187—188 | Fruit set |
| 4117 | $-CH_3$ | $-CH_3$ | 4-$CF_3$ | | 131—133 | |
| 4118 | $-CH_3$ | $-CH_3$ | 2-$CH_3$— 4-Cl | | 86—88 | Fruit set, tillers |

TABLE I
Part A (continued)

Compounds of the formula

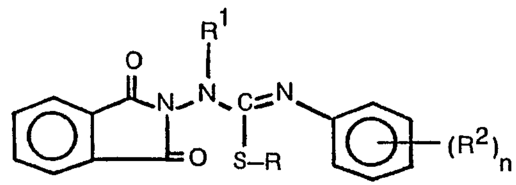

| Compnd. No. | R | $R^1$ | $(R^2)n$ | Salt Type | M.P. °C. | Comments on Utility |
|---|---|---|---|---|---|---|
| 4119 | $—CH_3$ | $—CH_3$ | $3,4-OCH_2O—$ | | 114—117 | Fruit set, tillers |
| 4120 | $—CH_3$ | $—CH_3$ | $2,4-Cl_2$ | | 84—86 | Fruit set, tillers |
| 4121 | $—C_2H_5$ | —H | $2,4-Cl_2$ | | 128—130 | Inc. fruit set, tillers |
| 4122 | $—CH_3$ | —H | 4-OEt | | 162—165 | Tillers |
| 4123 | $—C_2H_5$ | —H | 4-OEt | | 142—144 | |
| 4124 | $—CH_3$ | —H | —2-F | | 139—141 | Good fruit set |
| 4125 | $—C_2H_5$ | —H | 2-F | | 110—112 | Fruit set |
| 4126 | $—CH_3$ | $—CH_3$ | 4-OEt | | 114—116 | Dark green, tillers |
| 4127 | $—CH_3$ | $—CH_3$ | 2-F | | 87—89 | Fruit set, tillers |
| 4128 | $—C_2H_5$ | $—CH_3$ | $2,4-Cl_2$ | | 86—88 | Fruit set |
| 4129 | $—C_2H_5$ | $—CH_3$ | 4-OEt | | 102—106 | Fruit set |
| 4130 | $—C_2H_5$ | $—CH_3$ | 2-F | | 79—81 | Fruit set |
| 4259 | $—CH_3$ | $—CH_3$ | $3,4-CH_2CH_2CH_2—$ | | Semi-solid | Fruit set |

TABLE I
Part A (continued)

Compounds of the formula

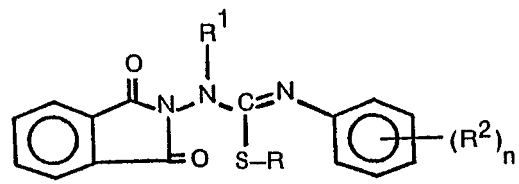

| Compnd. No. | R | $R^1$ | $(R^2)n$ | Salt Type | M.P. °C. | Comments on Utility |
|---|---|---|---|---|---|---|
| 4261 | $-CH_3$ | $-CH_3$ | 3-COOEt | | 143—146 | |
| 4262 | $-C_2H_5$ | $-CH_3$ | 3,4-$CH_2CH_2CH_2$— | | Oil | Fruit set |
| 4263 | $-C_2H_5$ | $-CH_3$ | 3-COOEt | | 98—101 | |
| 4264 | propyl | $-CH_3$ | 2,4-$Cl_2$ | | 85—87 | Fruit set |
| 4290 | $-CH_2CH{=}CH_2$ | $-CH_3$ | 4-$CF_3$ | | Oil | Fruit set |
| 4291 | 2-(2,4-dichloro-phenoxy) ethyl | $-CH_3$ | 2-F | | Oil | |
| 4292 | $-CH_2CH{=}CH_2$ | $-CH_3$ | 3,4-$OCH_2O$— | | Oil | |
| 4293 | 4-fluoro-benzoyl-methyl | —H | 4-F | | 98—101 | |
| 4329 | 1-benzoyl-ethyl | $-CH_3$ | 4-$OCH_3$ | | 80—83 | |
| 4330 | 4-fluoro-benzoyl-methyl | $-CH_3$ | 2-$CH_3$ | | 78—81 | Fruit set |
| 4331 | 4-fluoro-benzoyl-methyl | $-CH_3$ | 2-Cl | | 75—78 | Fruit set |

TABLE I
Part A (continued)

Compounds of the formula

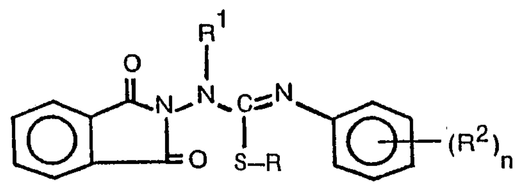

| Compnd. No. | R | $R^1$ | $(R^2)n$ | Salt Type | M.P. °C. | Comments on Utility |
|---|---|---|---|---|---|---|
| 4332 | 4-fluoro-benzoyl-methyl | $—CH_3$ | 2-F | | 60—63 | Fruit set oat tillers |
| 4333 | 2-(N-ethyl-N-phenyl-carbamyl) ethyl | —H | n = 0 | | 184—186 | Tillers |
| 4355 | $—CH_2COOH$ | $—CH_3$ | n = 0 | HBr | 115—120 | Tillers |
| 4373 | $—CH_3$ | 2-phenoxy-ethyl | n = 0 | | Glass | Fruit set tillers |
| 4374 | $—CH_3$ | $—CH_2CH_2OCH{=}CH_2$ | n = 0 | | 147—149 dec. | Fruit set |
| 4375 | $—CH_3$ | 3-chlorobenzyl | n = 0 | | Glass | Fruit set |
| 4429 | $—CH_3$ | isopropyl | n = 0 | | 116—118 | Fruit set |
| 4230 | $—C_2H_5$ | isopropyl | n = 0 | | 170—172 | |
| 4496 | 4-pyridyl-methyl | H | n = 0 | | 183—185 | |
| 4497 | 2-pyridyl-methyl | H | n = 0 | | 168—171 | |

TABLE I
Part A (continued)
Compounds of the formula

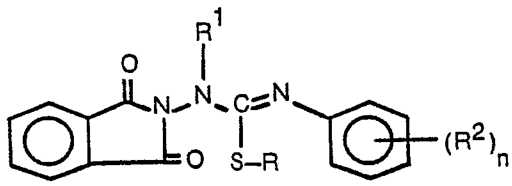

| Compnd. No. | R | $R^1$ | $(R^2)n$ | Salt Type | M.P.°C. | Comments on Utility |
|---|---|---|---|---|---|---|
| 4498 | 3-pyridyl-methyl | H | n=0 | | 202—205 | |
| 4499 | 4-pyridyl-methyl | H | 3-F | | 75—80 | |
| 4500 | 2-pyridyl-methyl | H | 3-F | | 195—197 | |
| 4501 | 3-pyridyl-methyl | H | 3-F | | 183—186 | Increased fruit set |
| 4502 | 4-pyridyl-methyl | $—CH_3$ | n=0 | | 110—115 | Increased fruit set |
| 4503 | 3-pyridyl-methyl | $—CH_3$ | n=0 | | 79—81 | Increased fruit set |
| 4504 | 2-pyridyl-methyl | $—CH_3$ | 3-F | | 109—112 | Increased fruit set |
| 4505 | 3-pyridyl-methyl | $—CH_3$ | 3-F | | 170—175 | Increased fruit set |
| 4455 | 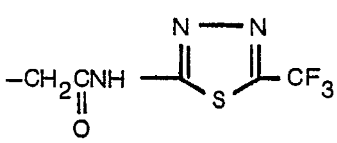 | —H | n=0 | | 213—215 | |
| 4456 | $—C(CH_3)_2$-benzoyl | —H | n=0 | | 193—196 | |
| 4457 | 3-phenyl-allyl | $—CH_3$ | n=0 | | Oil | Tillers |
| 4458 | 3-phenyl-allyl | —H | 3F | | Oil | Tillers |
| 4459 | $—C(CH_3)_2$-benzoyl | —H | 3F | | 150—153 | |
| 4460 | $—CH_2CH_2CF{=}CF_2$ | —H | n=0 | | 110—112 | |
| 4461 | 2-(N-ethyl-N-phenyl-carbamyl) ethyl | $—CH_3$ | 3-F | | Oil | Fruit set, tillers |
| 4462 | $—CH_2CH_2CF{=}CF_2$ | $—CH_3$ | 3-F | | 60—62 | Fruit set, tillers |

TABLE I
Part A (continued)

Compounds of the formula

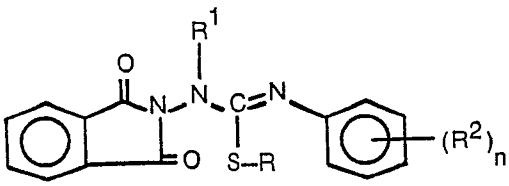

| Compnd. No. | R | $R^1$ | $(R^2)n$ | Salt Type | M.P.°C. | Comments on Utility |
|---|---|---|---|---|---|---|
| 4463 | $-CH_2CH_2CF{=}CF_2$ | $-CH_3$ | n = 0 | | Oil | Fruit set, tillers |
| 4313 | $-CH_3$ | $-CH_3$ | n = 0 | | 134—138 | |
| 4359 | $-CH_3$ | $-C_2H_5$ | n = 0 | | 114—116 | Fruit set |
| 4360 | $-C_2H_5$ | $-C_2H_5$ | n = 0 | | 107—108 | Fruit set |

TABLE I
Part A (continued)

Compounds of the formula

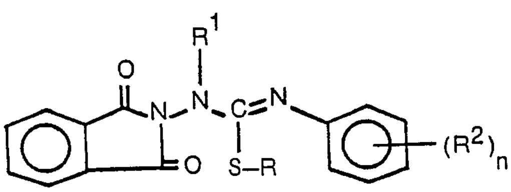

| Compnd. No. | R | $R^1$ | Ar $(R^2)n$ | M.P.°C. | Comments on Utility |
|---|---|---|---|---|---|
| 4258 | $-CH_3$ | $-CH_3$ | 3-methylbenzoyl | 169—172 | Increased fruit set |

TABLE Ia

PART B

EFFECTS ON PLANT SPECIES

| Compound No. | Preemergent Effects | | | | | | Postemergent Effects | | | | | |
|---|---|---|---|---|---|---|---|---|---|---|---|---|
| | Crab-grass | Cox-comb | Brome | Millet | Radish | Sugar Beet | Millet | Alfalfa | Oat | Radish | Sugar Beet | Tomato |
| 2878 | K4 | F3G3 | F3G3 | F3G3 | F3G2 | K4 | N2G3 | F3G2 | 0 | N2G1 | F2G2 | F3 |
| 2960 | F3G3 | F3G3 | F3G3 | F3G3 | F3G2 | K4 | F1 N2G2 | F2G2 | F1 N1G1 | F2 N1G2 | F1G2 | F2 |
| 2961 | K4 | F3G3 | F3G3 | F3G3 | F2G2 | K4 | N2G2 | F2G2 | F2G2 | F2G3 | F1G2 | F3G2 |
| 3062 | 0 | 0 | 0 | 0 | 0 | 0 | 0 | 0 | 0 | 0 | F2G2 | 0 |
| 3099 | F3G3 | F2G2 | F3G3 | F3G3 | F3G2 | K4 | F1G2 | F3G2 | F1G1 | F3G2 | F1G3 | N1F2 |
| 3100 | K4 | F2G2 | F3G3 | F3G3 | F2G2 | K4 | F2 N1G1 | F3G2 | N1 | F2 N1G2 | F2G2 | F3 |
| 3101 | F3G2 | F2G1 | F3G3 | F2G3 | F2G1 | F2G2 | F2 N1G1 | F2 N2G2 | N1F2 | F1 N1G2 | F2G2 | F2G1 |
| 3102 | F2G1 | F1G1 | F2 | F3G2 | F1G1 | F1 | F1 N1G1 | F2G1 | N1F1 | F2G2 | F2G2 | F3G1 |
| 3104 | F1G1 | 0 | F2 | F2 | F1 | F1 | N1 | F1 | N1G1 | F1 | F2G1 | F2 |
| 3105 | F3G3 | F2G1 | F3G3 | F3G3 | F3G2 | K4 | F3 N1G3 | F3G2 | N1F1 | F1G2 | F2G2 | F2 |
| 3110 | K4 | F2G1 | F3G2 | F3G3 | F2G2 | K4 | F1 N1G2 | F2G2 | N1G1 | F1G1 | F2G2 | F2 |
| 3111 | F3G3 | F2G1 | F3G3 | F3G3 | F2G1 | K4 | F2 N1G2 | F3G2 | F2 | F2G2 | F2G2 | F3G2 |
| 3129 | 0 | 0 | 0 | 0 | 0 | 0 | 0 | 0 | 0 | 0 | N1F1 | 0 |
| 3130 | | | | | | | | F2 | 0 | | F2 | F3 |
| 3134 | 0 | 0 | 0 | 0 | 0 | 0 | 0 | F1 | N1 | 0 | F2G1 | F2 |
| 3135 | F1G1 | 0 | F1 | F2G2 | G2 | 0 | F2G1 | F2 | F1 | F1 | F2G2 | F3 |

TABLE Ia

PART B (Continued)

EFFECTS ON PLANT SPECIES

| Compound No. | Preemergent Effects: Crab-grass | Cox-comb | Brome | Millet | Radish | Sugar Beet | Postemergent Effects: Millet | Alfalfa | Oat | Radish | Sugar Beet | Tomato |
|---|---|---|---|---|---|---|---|---|---|---|---|---|
| 3141 | K4 | F3G3 | K4 | K4 | F3G3 | K4 | F2 N3G3 | F3 N2G3 | F1G2 | F2 N2G3 | F2 N2G3 | F3 |
| 3143 | F3G3 | F2G2 | F3G3 | F3G3 | F2G2 | K4 | F2 N2G3 | F3G2 | F1 | F2G2 | F2G1 | F3G1 |
| 3144 | K4 | N4 | F3G3 | F3G3 | F2G2 | F2G1 | F1 N2G1 | F3G2 | N1F1 | F2 N1G2 | F2G2 | F3 |
| 3171 | 0 | 0 | 0 | 0 | 0 | 0 | 0 | F1 | N1 | F1 N1G1 | F1 | F2 |
| 3176 | F3G3 | F3G3 | F3G3 | F3G3 | F2G2 | F3G3 | F2 | F3G2 | N1F2 | F1 | F2G3 | F3 |
| 3191 | F1G1 | F1 | F3G1 | F3G3 | F3G2 | F3G2 | N1F1 | F2 | F1 | F2 | F2G2 | F2 |
| 3192 | 0 | 0 | 0 | 0 | 0 | 0 | 0 | 0 | 0 | 0 | F1 | 0 |
| 3193 | 0 | 0 | F2 | 0 | 0 | 0 | 0 | F1 | F1 | F1 | F1G3 | 0 |
| 3205 | K4 | K4 | F3G3 | F3G3 | F3G2 | F3G3 | F3 N3G3 | F3 G3 | F1G2 | F2G2 | F2G3 | F3 |
| 3206 | F3G3 | F3G3 | F3G3 | F3G3 | F3G2 | K4 | F2 N1G2 | F2G3 | 0 | F2G2 | F2G3 | F3 |
| 3215 | F3G2 | F2G1 | F3G3 | F3G2 | F2G2 | F3G3 | 0 | G2F3 | F1 | F2 | F2G2 | F3G1 |
| 3216 | F3G3 | F3G3 | K4 | F3G3 | F3G2 | F3G3 | F1 | F3 | F2 | F2 | F2G3 | F2 |
| 3218 | 0 | 0 | F1 | 0 | 0 | 0 | 0 | F2 N1G2 | 0 | N1 | F3G2 | 0 |
| 3219 | F1G1 | 0 | F2 | 0 | 0 | 0 | N1 | F3G2 | F1 | F1 | F2G2 | F1 |
| 3220 | F3G3 | F2G2 | K4 | F3G3 | F3G3 | F2G2 | F3 N1G2 | F3G2 | F1 | F1G1 | F3G3 | F3G1 |

TABLE Ia

PART B (Continued)

EFFECTS ON PLANT SPECIES

| Compound No. | Preemergent Effects | | | | | | Postemergent Effects | | | | | |
|---|---|---|---|---|---|---|---|---|---|---|---|---|
| | Crab-grass | Cox-comb | Brome | Millet | Radish | Sugar Beet | Millet | Alfalfa | Oat | Radish | Sugar Beet | Tomato |
| 3221 | F3G3 | F3G3 | F3G2 | F3G3 | F3G2 | F2G2 | F1 | F1G1 | F1 | F2 | F2G2 | F1G1 |
| 3222 | F1 | 0 | F1 | 0 | 0 | 0 | 0 | F2G1 | 0 | 0 | F1G2 | 0 |
| 3251 | 0 | 0 | 0 | 0 | 0 | 0 | 0 | F1 | 0 | 0 | 0 | 0 |
| 3274 | F1 | 0 | F2 | F2 | F1 | 0 | N1 | N1F1 | 0 | N1G1 | F2G3 | N1F1 |
| 3276 | 0 | 0 | F2 | F2 | 0 | 0 | 0 | F2 | 0 | F1 | F2G2 | 0 |
| 3277 | F3G3 | F2G2 | F3G3 | F3G3 | F3G2 | F3G2 | F1G1 | F3G3 | F1G1 | F2G1 | F2 N2G3 | F2 |
| 3278 | F2G1 | F2 | F2 | F2G1 | F1G1 | F1G1 | 0 | F2G1 | F1 | 0 | F3G2 | 0 |
| 3292 | F3G3 | F3G2 | F3G3 | F3G3 | F3G3 | F3G3 | N1G2 | F3G2 | F2 | F3G2 | F2G3 | F2 |
| 3293 | | | | | | | | | | | | |
| 3294 | F2G2 | F2G2 | F3G3 | F3G2 | F2G2 | K4 | F1G1 | F3G2 | F2 | F3G2 | F3G2 | F1 |
| 3295 | F2 | F1 | F2 | F1 | F1 | F1 | 0 | F3G2 | F1 | F2G2 | F3G3 | F2 |
| 3296 | 0 | 0 | F1 | F1 | 0 | 0 | 0 | F1 | 0 | 0 | F2G2 | 0 |
| 3297 | 0 | 0 | 0 | 0 | 0 | 0 | 0 | F1 | 0 | 0 | F1G1 | F1 |
| 3298 | | | | | | | | | | | | |

TABLE Ia

PART B (Continued)

EFFECTS ON PLANT SPECIES

| Compound No. | Preemergent Effects | | | | | | Postemergent Effects | | | | | |
|---|---|---|---|---|---|---|---|---|---|---|---|---|
| | Crab-grass | Cox-comb | Brome | Millet | Radish | Sugar Beet | Millet | Alfalfa | Oat | Radish | Sugar Beet | Tomato |
| 3283 | F1 | F1 | F2 | F2G2 | F1 | F1 | 0 | 0 | 0 | F1G1 | F2G3 | N1 |
| 3287 | 0 | F1 | F1 | F2G1 | F1G1 | F1G1 | 0 | 0 | F1 | 0 | F1G2 | N1 |
| 3409 | F3G3 | K4 | K4 | F3G3 | F3G3 | K4 | F2 N1G2 | F3G3 | F1 N1G1 | F3G3 | F3G2 | F3 |
| 3410 | F3G3 | K4 | K4 | F3G3 | F3G3 | K4 | F2 N1G2 | F3G3 | G2 | F2G2 | F3G3 | F3 |
| 3411 | F3G3 | K4 | K4 | F3G3 | K4 | K4 | N2G3 | F3G2 | G2F1 | F1 N2G3 | F2G3 | F2G3 |
| 3497 | F2G2 | F1 | F3G1 | F3G1 | F1G1 | F2G1 | 0 | F2G1 | 0 | N1 | F3G1 | F3 |
| 3500 | F1G1 | F2G1 | F3G2 | F2G2 | F2G2 | F2G2 | F1G1 | F2G1 | F1G1 | F1 | F3G1 | F2 |
| 3627 | F1G1 | K4 | F3G3 | F1G1 | F2G2 | F3G2 | 0 | F2G1 | F1G1 | F2G1 | F3G2 | F2 |
| 3628 | F3G3 | F3G3 | K4 | F3G3 | F3G2 | F3G3 | G2F1 | F3G3 | G2F1 | F2G1 | F3G2 | F3 |
| 3629 | 0 | 0 | F2 | 0 | 0 | 0 | 0 | F1 | 0 | F1 | F2 | F1 |
| 3631 | 0 | 0 | F2 | 0 | 0 | 0 | 0 | F1 | 0 | 0 | F2 | 0 |
| 3632 | F3G3 | F2G2 | K4 | F3G2 | F3G2 | F3G2 | G1 | F3 | G1 | F1G1 | F3G1 | F3G2 |
| 3633 | F3G2 | F2G1 | F3G3 | F2G1 | F3G2 | F2G1 | 0 | F2 | 0 | F1 | F3G1 | F3G1 |
| 3635 | 0 | F1 | F2 | 0 | F1 | F1 | 0 | F2 | 0 | F1 | F2G2 | F2 |
| 3637 | 0 | 0 | 0 | 0 | 0 | 0 | 0 | F2 | 0 | F1 | F2 | 0 |

TABLE Ia

PART B (Continued)

EFFECTS ON PLANT SPECIES

| Compound No. | Preemergent Effects | | | | | | Postemergent Effects | | | | | |
|---|---|---|---|---|---|---|---|---|---|---|---|---|
| | Crab-grass | Cox-comb | Brome | Millet | Radish | Sugar Beet | Millet | Alfalfa | Oat | Radish | Sugar Beet | Tomato |
| 3299 | F1 | F1 | F2 | F1 | F1 | F1 | 0 | F2G1 | 0 | F1 | F2G3 | 0 |
| 3300 | 0 | 0 | 0 | 0 | 0 | 0 | N1 | F1 | 0 | 0 | F2G3 | 0 |
| 3302 | 0 | 0 | 0 | 0 | 0 | 0 | 0 | F2 | 0 | 0 | F1G1 | 0 |
| 3303 | 0 | 0 | 0 | 0 | 0 | 0 | 0 | F2 | 0 | 0 | F1G1 | 0 |
| 3339 | 0 | 0 | 0 | 0 | 0 | 0 | 0 | 0 | 0 | 0 | F1 | F1 |
| 3348 | 0 | 0 | 0 | 0 | 0 | 0 | 0 | 0 | 0 | 0 | F1 | F1 |
| 3340 | 0 | 0 | 0 | 0 | 0 | 0 | 0 | 0 | 0 | 0 | F1 | F1 |
| 3342 | 0 | F1 | F2 | 0 | F1 | F1G1 | 0 | F2G1 | 0 | F1 | F3G3 | 0 |
| 3344 | F1G1 | F2G1 | F3G3 | F1 | F1G1 | F3G3 | 0 | F2 | 0 | F2 | F3G3 | F1 |
| 3346 | F3G3 | K4 | F3G3 | F3G2 | F2G2 | K4 | F2 | F3G2 | F2G1 | F2G1 | F2G3 | F2 |
| 3328 | 0 | 0 | 0 | 0 | N1 | 0 | 0 | 0 | 0 | 0 | F1 | 0 |
| 3329 | F2G2 | F3G3 | F3G3 | F3G3 | F3G2 | F2G2 | F1G2 | F3G2 | F1 | N1F2 | F3G2 | N1F1 |
| 3331 | K4 | F3G3 | F3G3 | K4 | F3G3 | F3G3 | N2G3 | F3G3 | F1 | F2G2 | F3G2 | F1 |
| 3332 | 0 | 0 | 0 | 0 | 0 | 0 | 0 | 0 | 0 | 0 | F1 | 0 |

TABLE Ia

PART B (Continued)

EFFECTS ON PLANT SPECIES

| Compound No. | Preemergent Effects | | | | | | Postemergent Effects | | | | | |
|---|---|---|---|---|---|---|---|---|---|---|---|---|
| | Crab-grass | Cox-comb | Brome | Millet | Radish | Sugar Beet | Millet | Alfalfa | Oat | Radish | Sugar Beet | Tomato |
| 3700 | 0 | 0 | F1 | 0 | 0 | 0 | 0 | F1 | F2 | F2G2 | F3G2 | F2 |
| 3701 | 0 | 0 | 0 | 0 | 0 | 0 | 0 | G2F1 | 0 | F1 | F2 | N1 |
| 3702 | 0 | 0 | F1 | 0 | 0 | 0 | 0 | F1 | F1 | F1 | F3 | N1 |
| 3703 | 0 | 0 | 0 | 0 | 0 | 0 | 0 | F2G2 | F1 | 0 | F3 | 0 |
| 3704 | 0 | 0 | 0 | 0 | 0 | 0 | 0 | 0 | — | 0 | 0 | F2 N1G1 |
| 3705 | 0 | 0 | 0 | 0 | 0 | 0 | N1 | F2 | 0 | F1 | N1F1 | F1 |
| 3706 | 0 | 0 | 0 | 0 | 0 | 0 | 0 | F2 | 0 | F1 | F3G1 | F1 |
| 3707 | 0 | 0 | 0 | 0 | 0 | 0 | 0 | F1 | 0 | F1 | F2G2 | F2 |
| 3708 | 0 | 0 | 0 | 0 | 0 | 0 | 0 | 0 | 0 | F1 | F2 | 0 |
| 3709 | F2G1 | F2G1 | F2 | F1G1 | F3G3 | F2G1 | 0 | F2G1 | 0 | F1 | F3G2 | F2 |
| 3710 | F3G3 | F3G2 | F3G3 | F3G2 | F3G3 | F3G3 | F2G1 | F3G1 | F2 | F2G2 | F3G2 | F2G1 |
| 3731 | 0 | F1 | F2 | 0 | G1 | F2 | 0 | F2 | F1 | F1 | F3 | N1F2 |

TABLE Ia

PART B (Continued)

EFFECTS ON PLANT SPECIES

| Compound No. | Preemergent Effects | | | | | | Postemergent Effects | | | | | |
|---|---|---|---|---|---|---|---|---|---|---|---|---|
| | Crab-grass | Cox-comb | Brome | Millet | Radish | Sugar Beet | Millet | Alfalfa | Oat | Radish | Sugar Beet | Tomato |
| 3732 | 0 | 0 | 0 | 0 | 0 | 0 | 0 | 0 | 0 | 0 | F2 | 0 |
| 3733 | 0 | 0 | 0 | 0 | 0 | 0 | 0 | F1 | 0 | F2 | F3G1 | F2 |
| 3734 | 0 | 0 | 0 | 0 | 0 | 0 | 0 | 0 | 0 | 0 | F1 | 0 |
| 3749 | 0 | 0 | 0 | 0 | 0 | 0 | 0 | 0 | 0 | 0 | F3G1 | 0 |
| 3750 | F1 | F1 | F3G3 | F2G2 | F1 | F1 | 0 | F2G2 | F1N1 | F2G1 | F3G1 | F2G2 |
| 3751 | 0 | 0 | 0 | 0 | 0 | 0 | 0 | F1 | 0 | 0 | F1 | N1 |
| 3752 | K4 | F1G1 | K4 | F2G2 | F2G2 | K4 | F1G3 | F2G3 | G1 | F2 | F2G1 | F3 |
| 3780 | F1 | F2G1 | F3G3 | F2G1 | F2G2 | F2G1 | N1 | F2G2 | N1 | F2<br>N1G2 | F3G1 | F1 |
| 3781 | F2G1 | – | K4 | F2G2 | F3G2 | F2G2 | 0 | F3G2 | F2 | F2<br>N2G2 | F3 | N1F2 |
| 3782 | 0 | F1 | F2 | 0 | 0 | F1 | 0 | F2 | 0 | F2 | F3 | N1F1 |
| 3783 | F1 | F1 | F3G2 | F2G2 | F2G1 | F2 | N1G1 | N1 | G1 | F1G1 | F2G2 | F2 |
| 3784 | 0 | 0 | F1 | 0 | 0 | 0 | 0 | 0 | 0 | 0 | F2G1 | F1 |

TABLE Ia

PART B (Continued)

EFFECTS ON PLANT SPECIES

| Compound No. | Preemergent Effects | | | | | | Postemergent Effects | | | | | |
|---|---|---|---|---|---|---|---|---|---|---|---|---|
| | Crab-grass | Cox-comb | Brome | Millet | Radish | Sugar Beet | Millet | Alfalfa | Oat | Radish | Sugar Beet | Tomato |
| 3785 | F2G1 | F2G2 | F3G3 | F3G2 | F3G3 | F3G2 | N1G2 | F2G2 | G1 | F2G2 | F3G2 | F2G1 |
| 3786 | F1G1 | F3G3 | F3G3 | F2G2 | F3G3 | F3G2 | F1G1 | F1 | – | F2 | F3G1 | F2 |
| 3787 | 0 | 0 | F1 | 0 | 0 | 0 | N1 | 0 | 0 | F1 | F2G1 | N1 |
| 3788 | F3G3 | K4 | K4 | F3G2 | K4 | K4 | F3G2 | F2G3 | N1G2 | F2 N1G3 | F1 N1G2 | F2 |
| 3789 | 0 | F1 | F1 | 0 | 0 | 0 | 0 | F1 | 0 | 0 | F2G1 | 0 |
| 3790 | 0 | 0 | F2 | 0 | 0 | 0 | 0 | F3 | 0 | 0 | F1G2 | N1F1 |
| 3791 | F2G1 | F2G2 | F3G2 | F2G2 | F2G2 | F3G2 | 0 | F1 | G1 | N1G1 | F2G2 | F2G1 |
| 3792 | F1G1 | F2G1 | F3G3 | F3G3 | F3G3 | K4 | F2G2 | F2G3 | G1F1 | F3G3 | F2G2 | F2G2 |
| 3793 | 0 | 0 | F1 | 0 | 0 | F1 | 0 | 0 | 0 | F1G1 | F2G1 | 0 |
| 3794 | 0 | 0 | F2 | 0 | 0 | F1 | 0 | F1 | 0 | F1 | F1G1 | 0 |
| 3795 | 0 | 0 | 0 | 0 | 0 | 0 | 0 | 0 | 0 | 0 | F1 | F1 |
| 3796 | 0 | 0 | K3G3 | 0 | 0 | F1 | 0 | 0 | 0 | F2 | F2G2 | F2 |

TABLE Ia

PART B (Continued)

EFFECTS ON PLANT SPECIES

| Compound No. | Preemergent Effects | | | | | | Postemergent Effects | | | | | |
|---|---|---|---|---|---|---|---|---|---|---|---|---|
| | Crab-grass | Cox-comb | Brome | Millet | Radish | Sugar Beet | Millet | Alfalfa | Oat | Radish | Sugar Beet | Tomato |
| 3797 | 0 | 0 | F1 | 0 | 0 | K3G3 | 0 | 0 | 0 | 0 | F1 | 0 |
| 3798 | 0 | 0 | 0 | 0 | 0 | 0 | 0 | 0 | 0 | 0 | F1 | 0 |
| 3799 | G1 | 0 | N3G1 | 0 | 0 | 0 | 0 | 0 | 0 | F1 | F1 | 0 |
| 3800 | 0 | 0 | F2 | 0 | 0 | F1 | 0 | 0 | 0 | 0 | – | N1 |
| 3812 | F2G2 | F3G3 | K4 | F2G2 | F2G2 | F3G3 | F1G1 | F3G1 | F1G1 | F1 | F3G2 | F2 |
| 3813 | 0 | F1 | F2 | 0 | 0 | F1 | 0 | F2 | 0 | F1 | F2 | 0 |
| 3814 | F3G3 | K4 | K4 | F3G2 | F3G3 | F3G3 | F2G2 | F3G3 | F2G2 | F3G2 | F3G3 | F3G1 |
| 3815 | K4 | K4 | K4 | F3G3 | F3G3 | K4 | G1 | F3G2 | F1G1 | F1 | F3G1 | F2 |
| 3816 | 0 | – | F2G1 | 0 | 0 | 0 | 0 | F1 | 0 | 0 | 0 | 0 |
| 3849 | F2G2 | F3G2 | F3G3 | F1G2 | F2G2 | F2G2 | F3G2 | F3G3 | F2G2 | F2G2 | F3G3 | F3G1 |
| 3850 | 0 | F1G1 | F3G1 | G1 | 0 | F1 | F1 | F3 | F1 | F1 | F2G1 | F3 |
| 3851 | 0 | 0 | 0 | 0 | 0 | 0 | 0 | F1 | 0 | 0 | F1 | F1 |

TABLE Ia

PART B (Continued)

EFFECTS ON PLANT SPECIES

| Compound No. | Preemergent Effects | | | | | | Postemergent Effects | | | | | |
|---|---|---|---|---|---|---|---|---|---|---|---|---|
| | Crab-grass | Cox-comb | Brome | Millet | Radish | Sugar Beet | Millet | Alfalfa | Oat | Radish | Sugar Beet | Tomato |
| 3977 | 0 | F1 | F2G1 | 0 | F1 | F2 | 0 | F2 | N2G2 | F1 | F3 | F3 |
| 4017 | 0 | 0 | 0 | 0 | 0 | 0 | 0 | F2 | 0 | 0 | F1 | F1 |
| 4019 | 0 | 0 | 0 | 0 | 0 | F1 | 0 | F1 | 0 | 0 | F1 | F1 |
| 4020 | 0 | F1 | 0 | 0 | 0 | F1 | 0 | 0 | 0 | 0 | F1 | F1 |
| 4021 | 0 | – | 0 | N1 | 0 | N1 | 0 | 0 | 0 | 0 | 0 | 0 |
| 4023 | F1 | F2G2 | F2G1 | F1G1 | F2G1 | F3G2 | F1 | F2G1 | F2G1 | F3G1 | F3G1 | F2 |
| 4025 | 0 | F1 | F2 | 0 | 0 | F1 | F1G1 | F2G1 | F1 | F1 | F2G2 | F1 |
| 4026 | 0 | F1 | F2 | 0 | 0 | G2F2 | F1 | F2 | F1 | F2 | F1 | F2 |
| 4027 | F2G2 | N4 | N4 | F2G2 | F2G1 | F3G1 | F2G1 | F3G1 | F1G1 | F2G1 | F3G2 | F3E1 |
| 4028 | 0 | 0 | F2 | 0 | F1 | F2 | F1 | F3G1 | F1 | F2 | F2G1 | F1 |
| 4029 | 0 | 0 | F1 | 0 | 0 | F1 | 0 | 0 | 0 | 0 | 0 | 0 |

TABLE Ia

PART B (Continued)

EFFECTS ON PLANT SPECIES

| Compound No. | Preemergent Effects | | | | | | Postemergent Effects | | | | | |
|---|---|---|---|---|---|---|---|---|---|---|---|---|
| | Crab-grass | Cox-comb | Brome | Millet | Radish | Sugar Beet | Millet | Alfalfa | Oat | Radish | Sugar Beet | Tomato |
| 4030 | F1G1 | F2G2 | F3G3 | F1G1 | F3G3 | F3G3 | F2G1 | F3G1 | F2G2 | F3G2 | F3G2 | F3G1 |
| 4033 | 0 | 0 | 0 | 0 | 0 | 0 | 0 | F1 | 0 | 0 | F1 | F1 |
| 4034 | 0 | 0 | 0 | 0 | 0 | 0 | 0 | 0 | 0 | 0 | F1 | F1 |
| 4035 | 0 | 0 | 0 | 0 | 0 | F1 | 0 | F1 | 0 | 0 | F1 | F1 |
| 4036 | 0 | 0 | 0 | 0 | 0 | 0 | 0 | 0 | 0 | 0 | N2F1 | F1 |
| 4062 | F2G2 | F3G2 | F3G3 | F3G2 | F3G2 | '3G3 | F1 | F3G2 | F1 | F3G2 | F2G1 | F3E1 |
| 4063 | F3G3 | F3G2 | K4 | F3G3 | F3G3 | F3G2 | F2G2 | F3G2 | F2G1 | F2G2 | F3G3 | F3G1 |
| 4064 | F3G3 | N4 | F3G2 | F2G1 | F2G1 | F2G1 | F1G1 | F3G2 | F1G1 | F2G2 | F3G2 | F3G1 |
| 4065 | 0 | 0 | 0 | 0 | 0 | 0 | 0 | F1 | 0 | 0 | 0 | 0 |
| 4066 | 0 | F1 | F2 | F1 | F1 | F2G1 | 0 | F1 | 0 | 0 | F1 | 0 |

TABLE Ia

PART B (Continued)

EFFECTS ON PLANT SPECIES

| Compound No. | Preemergent Effects | | | | | | Postemergent Effects | | | | | |
|---|---|---|---|---|---|---|---|---|---|---|---|---|
| | Crab-grass | Cox-comb | Brome | Millet | Radish | Sugar Beet | Millet | Alfalfa | Oat | Radish | Sugar Beet | Tomato |
| 4068 | F2G2 | F1G1 | F2 | F1G1 | G1 | F1G1 | 0 | F2 | F1 | F1 | F1 | F3 |
| 4070 | F1G1 | 0 | F1 | 0 | 0 | F1 | 0 | F1 | 0 | 0 | F1 | F1 |
| 4115 | F1G1 | F1 | F2 | F2G1 | F2G2 | F3G1 | F1 | F1 | F1 | F1 | F1 | F2 |
| 4116 | 0 | 0 | 0 | 0 | 0 | F1 | 0 | 0 | 0 | F1 | F1 | F1 |
| 4117 | F2G1 | F2 | F2G1 | F2G2 | F2G2 | F2G1 | F1G1 | F2G2 | F1 | F2G1 | N4 | F3 |
| 4118 | F2G2 | F2G1 | F3G2 | F3G2 | F2G2 | F2G2 | F2G2 | F2G1 | F2G1 | F2 | F2G1 | F3 |
| 4119 | G1 | F1 | F1 | 0 | F1 | F1 | F1G1 | F3G2 | F2G1 | F2 | F3G2 | F3G1 |
| 4120 | F1G1 | F1 | F3G1 | F2G1 | F2G1 | F3G2 | F2G1 | F3G2 | F2G1 | F2G2 | F3G2 | F3 |
| 4121 | 0 | F1 | F1G1 | 0 | F1 | F2G1 | F1 | F2 | F1G1 | F1 | F2 | F2 |
| 4122 | G1 | F1 | F2G1 | 0 | 0 | F2G1 | F1G1 | F3G2 | F1G1 | F1 | F2 | F3G1 |
| 4123 | 0 | 0 | 0 | 0 | 0 | 0 | 0 | 0 | 0 | 0 | F1 | 0 |
| 4124 | 0 | 0 | F1 | 0 | 0 | F1 | 0 | F1 | F1 | F1 | F1 | F1 |
| 4125 | 0 | 0 | 0 | 0 | 0 | 0 | 0 | F1 | F1 | 0 | F1 | F1 |
| 4126 | F1 | F2 | F3G1 | F2G1 | F2G1 | F3G1 | F1G1 | F3G1 | F2G1 | F2G2 | F1G1 | F3G3 |

TABLE Ia

PART B (Continued)

EFFECTS ON PLANT SPECIES

| Compound No. | Preemergent Effects: Crab-grass | Cox-comb | Brome | Millet | Radish | Sugar Beet | Postemergent Effects: Millet | Alfalfa | Oat | Radish | Sugar Beet | Tomato |
|---|---|---|---|---|---|---|---|---|---|---|---|---|
| 4127 | F2G2 | F3G2 | F3G3 | F3G2 | F3G3 | F2G1 | F2G2 | F3G2 | F2G1 | F1G1 | F2G1 | F3G1 |
| 4128 | F1G1 | F2G1 | F3G2 | F3G2 | F2G2 | F2G1 | F1G1 | F2G1 | F1 | F2G2 | F2G1 | F3 |
| 4129 | F2G1 | F3G3 | F3G3 | F2G1 | F2G2 | F2G2 | F1 | F2 | 0 | F1 | F2 | F3 |
| 4130 | F2G2 | F3G2 | F3G3 | F3G2 | F3G3 | F3G3 | F2G2 | F2G1 | F1 | F2G1 | F2G2 | F3 |
| 4259 | 0 | 0 | F1 | 0 | 0 | 0 | F1G1 | F2G1 | F1 | F1G1 | F2 | F3G1 |
| 4261 | 0 | 0 | 0 | 0 | 0 | 0 | 0 | 0 | 0 | 0 | F1 | 0 |
| 4262 | 0 | F1 | F2 | F1G1 | F2 | F2 | 0 | F1 | 0 | F1G1 | F2G1 | F3 |
| 4263 | 0 | 0 | 0 | 0 | 0 | 0 | 0 | 0 | – | 0 | F1 | 0 |
| 4264 | 0 | F1 | F3 | F1 | F1 | F2 | F1G1 | F2G1 | F1 | F1G1 | F3G1 | F3G1 |
| 4290 | F3G2 | F2G2 | F3G3 | F3G2 | F2G2 | F3G2 | F2G1 | F3G2 | – | F2G2 | F3G3 | F3G3 |
| 4291 | F3G3 | F3G3 | F3G2 | F3G3 | K4 | K4 | F3G3 | F3G3 | – | GW2 F3G3 | F3G3 | F3E3 |
| 4292 | 0 | F1 | F1 | F1 | 0 | 0 | F1 | F3G2 | F1 | F1 | F2G2 | F3 |
| 4293 | 0 | 0 | F1 | 0 | 0 | 0 | 0 | F1 | 0 | 0 | F1 | 0 |

TABLE Ia

PART B (Continued)

EFFECTS ON PLANT SPECIES

| Compound No. | Preemergent Effects | | | | | | Postemergent Effects | | | | | |
|---|---|---|---|---|---|---|---|---|---|---|---|---|
| | Crab-grass | Cox-comb | Brome | Millet | Radish | Sugar Beet | Millet | Alfalfa | Oat | Radish | Sugar Beet | Tomato |
| 4329 | F2G1 | K4 | F2G1 | F1G1 | F1 | F3G1 | N2G1 | F3G2 | F1 | F1 | F2 | F3 |
| 4330 | F3G2 | F2 | F3G1 | F3G2 | F2G1 | F3G1 | F1G1 | F2 | F1 | F1 | F2 | F3 |
| 4331 | F2G2 | F1 | F3G1 | F2G1 | F1 | F2G1 | N1G1 | F1 | F1 | F1 | F2 | F2 |
| 4332 | F3G2 | F2G1 | F3G2 | F3G2 | F3G3 | F3G3 | F2G2 | F3G3 | F2G1 | F2 | F3G2 | F3 |
| 4333 | F2G1 | F2 | F3G1 | F2G1 | F1 | F2G1 | F1G1 | F3G2 | F1 | F2 | F2G1 | F1 |
| 4355 | F3G3 | F3G3 | F3G3 | F3G2 | F3G2 | F3G3 | N2G2 | F3G3 | F1 | N1G1 | N4 | F2N1 |
| 4373 | F1 | F2G2 | F2 | F2G1 | 0 | F2G1 | 0 | F1 | F1 | F1 | F1G1 | F2 |
| 4374 | G1 | G1 | 0 | 0 | 0 | F2 | 0 | F1 | 0 | 0 | F1 | F1 |
| 4375 | 0 | F2 | F1 | F1 | F1G1 | F2G1 | N1 | F2 | 0 | G1 N2F1 | F3G2 | F2 |

TABLE Ia

PART B (Continued)

EFFECTS ON PLANT SPECIES

| Compound No. | Preemergent Effects | | | | | | Postemergent Effects | | | | | |
|---|---|---|---|---|---|---|---|---|---|---|---|---|
| | Crab-grass | Cox-comb | Brome | Millet | Radish | Sugar Beet | Millet | Alfalfa | Oat | Radish | Sugar Beet | Tomato |
| 4429 | 0 | F1 | 0 | F1 | F2 | F2 | | | | | | |
| 4230 | 0 | 0 | 0 | 0 | 0 | 0 | 0 | 0 | 0 | 0 | 0 | F1 |
| 4496 | 0 | F1 | F1 | 0 | 0 | F1 | N1 | F1 | 0 | N1 | F1 | F1 |
| 4497 | 0 | 0 | 0 | 0 | 0 | 0 | 0 | F3G2 | F1 | F1 | F2G1 | F1 |
| 4498 | 0 | 0 | 0 | 0 | 0 | 0 | 0 | 0 | 0 | F1 | F1 | F1 |
| 4499 | 0 | 0 | 0 | 0 | 0 | 0 | N1F1 | F3G3 | F1 | F1 | F1 | F1 |
| 4500 | 0 | 0 | 0 | 0 | 0 | 0 | 0 | 0 | 0 | 0 | F1 | F2 |
| 4501 | 0 | 0 | F1 | 0 | 0 | 0 | F1 | F3G2 | F1 | F1 | F2G1 | F2 |
| 4502 | F2G2 | F2G2 | F3G2 | F3G2 | F2G2 | F1G1 | F2G1 | F3G3 | F2 | F2 | F2G1 | F3 |
| 4503 | K4 | F3G3 | K4 | F3G3 | F3G3 | K4 | F3G2 | F3G3 | F2G1 | F3G3 | F3G2 | F3 |
| 4504 | K4 | K4 | F3G3 | F3G3 | F3G3 | F3G3 | F2G2 | F3G3 | F2G1 | F2G2 | F2G1 | F3 |
| 4505 | F3G3 | K4 | F3G3 | F3G2 | F3G2 | F3G2 | F2G2 | F3G3 | F2G1 | F2G1 | F3G1 | F3 |

TABLE Ia

PART B (Continued)

EFFECTS ON PLANT SPECIES

| Compound No. | Preemergent Effects | | | | | | Postemergent Effects | | | | | |
|---|---|---|---|---|---|---|---|---|---|---|---|---|
| | Crab-grass | Cox-comb | Brome | Millet | Radish | Sugar Beet | Millet | Alfalfa | Oat | Radish | Sugar Beet | Tomato |
| 4455 | 0 | F1 | 0 | F1 | F1 | 0 | | | | | | |
| 4456 | 0 | F2G2 | 0 | F1 | F1 | F1 | | | | | | |
| 4457 | F2G1 | F3G2 | F2 | F2 | F2G1 | F3 | | | | | | |
| 4458 | F2G1 | F3G3 | F2 | F1 | F2 | F3 | | | | | | |
| 4459 | 0 | F1 | 0 | F1 | F2 | F1 | | | | | | |
| 4460 | N1G1 | F2G1 | 0 | F1 | F1 | 0 | | | | | | |
| 4461 | N2G2 | F3G3 | F2 | F3G2 | F3G2 | F3G1 | | | | | | |
| 4462 | N1G1 | F3G3 | F2 | F2 | F3G2 | F2 | | | | | | |
| 4463 | N1G1 | F3G3 | F2G1 | F2G1 | F3G2 | F3G1 | | | | | | |
| 4313 | F1G1 | G1 | 0 | F1G1 | 0 | F2G1 | 0 | 0 | 0 | 0 | F1 | 0 |
| 4359 | F1 | – | F2 | F2G2 | F1 | F3G1 | 0 | F1G1 | 0 | F1G1 | F1G1 | F1 |
| 4360 | 0 | F1 | F1 | F2G1 | 0 | F1 | 0 | F1 | 0 | F1 | F1 | F1 |
| 4528 | 0 | 0 | F1 | 0 | 0 | 0 | F1G1 | F2G1 | F1 | F1G1 | F2 | F3G1 |

The use of many of the growth regulator compounds may be demonstrated by treatment of soybeans (*Soja max*) to increase the number of seed pods and by treating tomato plants (*Lycopersicum esculentum*) to increase fruit set. In an illustrative experiment, *Soja max* (Evans variety) and *Lycopersicum esculentum* (Tiny Tim variety) were grown in 4-inch pots (one plant per pot) filled with greenhouse potting soil (2 parts good top soil, 1 1/2 parts builder's sand, 1 1/2 parts peat, fertilized with 5 lb. of 12—12—6 fertilizer and 5 lb. of finely ground limestone per cu. yd.). Aqueous spray formulations were prepared and the potted plants were sprayed at a spray volume of 40 gal. per acre and at application rates of 16, 4, 1 and 1/4 oz. per acre. The spray mixtures were prepared by dissolving the proper amount of growth regulator compound in 15 ml. of acetone, adding 2 ml. of a solvent-emulsifier mixture consisting of 60 wt. percent of a commercial polyoxyethylated vegetable oil emulsifier (96 wt. percent active ingredient, Emulphor EL—719), 20 wt. percent xylene and 20 wt. percent deodorized kerosene, then bringing total volume up to 80 ml by addition of a 0.156 wt. percent aqueous solution of liquid non-ionic dispersant (90 wt. percent active trimethylnonyl polyethylene glycol ether, Tergitol TMN—10). Two replicates were sprayed at all application rates. For comparative purposes, plants were also sprayed at 40 gal./acre with water. The number of seed pods and of fruit as percentage of arithmetic mean of the numbers on untreated plants was observed within approximately three weeks after spray treatment and the results are tabulated below. The extent of growth regulatory effect on the plants was estimated on a scale of 0 to 10 and is also recorded in the following table:

GROWTH REGULATING EFFECTS ON TWO SPECIES

| Comp'd. No. | Rate oz/A | *Soja max* Pod Count① Percent in Comparison to Untreated Plants | *Soja max* Severity of Growth Regulating Effect② | *Lycopersicum esculentum* Fruit Count Percent in Comparison to Untreated Plants | *Lycopersicum esculentum* Severity of Growth Regulating Effect② |
|---|---|---|---|---|---|
| 2878 | 16 | 163 a,c | 9 | 73 a,b | 9 |
| | 4 | 183 a,c | 6.5 | 364 a,b | 9 |
| | 1 | 177 | 3 | 327 | 4 |
| 2960 | 16 | 160 a,c | 9 | 218 a | 9 |
| | 4 | 138 | 6.5 | 218 e | 7 |
| | 1 | 135 | 6.5 | 327 | 2 |
| 3099 | 16 | 183 a,c | 9 | 182 b | 9 |
| | 4 | 177 a,c | 6.5 | 400 b | 8 |
| | 1 | 186 | 2.5 | 436 b | 4.5 |
| 3100 | 16 | 166 a,c | 9 | 82 a | 9 |
| | 4 | 166 | 6.5 | 382 e | 6.5 |
| | 1 | 146 | 4 | 355 | 4 |
| 3105 | 16 | 185 a,c | 9 | 136 | 9 |
| | 4 | 171 | 7 | 382 | 6.5 |
| | 1 | 143 | 4 | 355 | 3 |
| 3143 | 16 | 166 a,c | 9 | 245 a | 9 |
| | 4 | 169 | 6 | 191 d | 7.5 |
| | 1 | 157 | 2 | 191 e | 3.5 |
| 3144 | 16 | 199 a,c | 9 | 55 a | 9 |
| | 4 | 157 | 5.5 | 382 d | 7 |
| | 1 | 157 | 2.5 | 245 e | 5 |
| 3176 | 16 | 111 | 2 | 178 b | 7.5 |
| | 4 | 104 | 0 | 141 | 3 |
| | 1 | 93 | 0 | 131 | 1 |
| 3251 | 16 | 86 | 0 | 129 | 0.5 |
| | 4 | 95 | 0 | 64 | 0 |
| | 1 | 91 | 0 | 64 | 0 |

GROWTH REGULATING EFFECTS ON TWO SPECIES (continued)

| | | *Soja max* | | *Lycopersicum esculentum* | |
|---|---|---|---|---|---|
| Comp'd. No. | Rate oz/A | Pod Count① Percent in Comparison to Untreated Plants | Severity of Growth Regulating Effect② | Fruit Count Percent in Comparison to Untreated Plants | Severity of Growth Regulating Effect② |
| 3274 | 16 | 114 | 0.5 | 107 | 0 |
| | 4 | 105 | 1 | 150 | 0 |
| | 1 | 86 | 0 | 86 | 0 |
| 3277 | 16 | 146 | 6.5 | 197 b | 8.5 |
| | 4 | 143 | 4.5 | 169 | 5.5 |
| | 1 | 154 | 2 | 122 | 1.5 |
| 3278 | 16 | 95 | 0 | 150 | 0.5 |
| | 4 | 91 | 0 | 107 | 0 |
| | 1 | 100 | 0 | 64 | 0 |
| 3293 | 16 | 154 | 7.5 | 141 b | 8 |
| | 4 | 150 | 3.5 | 178 | 4.5 |
| | 1 | 121 | 1 | 113 | 2.5 |
| 3294 | 16 | 154 a | 7.5 | 197 d | 7.5 |
| | 4 | 121 c | 4.5 | 169 b | 3.5 |
| | 1 | 154 | 1.5 | 122 | 2.5 |
| 3295 | 16 | 136 | 8 | 216 | 8 |
| | 4 | 175 | 4 | 188 | 3 |
| | 1 | 145 | 2 | 169 | 1.5 |
| 3297 | 16 | 139 | 1 | 113 | 0.5 |
| | 4 | 132 | 0 | 122 | 0 |
| | 1 | 114 | 0 | 84 | 0 |
| 3298 | 16 | 107 | 0.5 | 113 | 2 |
| | 4 | 118 | 0 | 141 | 0.5 |
| | 1 | 107 | 0 | 103 | 0 |
| 3299 | 16 | 118 | 1 | 279 | 2 |
| | 4 | 109 | 0.5 | 64 | 0 |
| | 1 | 86 | 0 | 21 | 0 |
| 3300 | 16 | 109 | 1 | 86 | 0 |
| | 4 | 114 | 0 | 129 | 0 |
| | 1 | 86 | 0 | 107 | 0 |
| 3302 | 16 | 86 | 1.5 | 107 | 1.5 |
| | 4 | 127 | 0.5 | 107 | 0 |
| | 1 | 118 | 0 | 86 | 0 |
| 3303 | 16 | 118 | 1.5 | 193 | 0.5 |
| | 4 | 123 | 1 | 43 | 0 |
| | 1 | 123 | 0 | 107 | 0 |
| 3339 | 16 | 107 | 0 | 94 | 0 |
| | 4 | 111 | 0 | 131 | 0 |
| | 1 | 114 | 0 | 103 | 0 |
| 3340 | 16 | 105 | 0 | 150 | 0 |
| | 4 | 105 | 0 | 171 | 0 |
| | 1 | 114 | 0 | 193 | 0 |

GROWTH REGULATING EFFECTS ON TWO SPECIES (continued)

| | | *Soja max* | | *Lycopersicum esculentum* | |
|---|---|---|---|---|---|
| Comp'd. No. | Rate oz/A | Pod Count① Percent in Comparison to Untreated Plants | Severity of Growth Regulating Effect② | Fruit Count Percent in Comparison to Untreated Plants | Severity of Growth Regulating Effect② |
| 3342 | 16 | 140 | 3 | 400 | 0 |
| | 4 | 105 | 0.5 | 200 | 0 |
| | 1 | 94 | 0 | 0 | 0 |
| 3344 | 16 | 136 c | 6 | 364 | 4 |
| | 4 | 145 | 3 | 321 | 2.5 |
| | 1 | 114 | 1.5 | 300 | 2 |
| 3345 | 16 | 143 | 5 | 169 b | 4.5 |
| | 4 | 164 | 3.5 | 188 | 2.5 |
| | 1 | — | 1 | 159 | 1 |
| 3346 | 16 | 159 a | 7 | 300 a | 8.5 |
| | 4 | 141 c | 5 | 279 b | 8 |
| | 1 | 123 | 2.5 | 364 | 4.5 |
| 3410 | 16 | 114a ① | 7.5 | 407 a,d | 8 |
| | 4 | 141 c① | 5.5 | 343 b,d | 7.5 |
| | 1 | 105 ① | 3 | 321 | 5 |
| 3497 | 16 | 121 | 0.5 | 141 | 1 |
| | 4 | 111 | 0 | 103 | 0 |
| | 1 | 111 | 0 | 122 | 0 |
| 3627 | 16 | 121 | 4.5 | 159 | 2 |
| | 4 | 104 | 2.5 | 141 | 2.5 |
| | 1 | 100 | 1 | 141 | 1.5 |
| 3628 | 16 | 125 a | 6.5 | 159 b | 8 |
| | 4 | 150 c | 6 | 188 d | 7.5 |
| | 1 | 136 | 2.5 | 169 | 3.5 |
| 3631 | 16 | 129 | 1.5 | 122 | 0.5 |
| | 4 | 104 | 0 | 84 | 0 |
| | 1 | 107 | 0 | 103 | 0 |
| 3632 | 16 | 132 a | 7.5 | 178 b | 7.5 |
| | 4 | 146 | 4.5 | 216 d | 6 |
| | 1 | 143 | 2.5 | 169 | 4.5 |
| 3633 | 16 | 143 | 2.5 | 700 | 3 |
| | 4 | 129 | 0.5 | 400 | 0.5 |
| | 1 | 112 | 0 | 0 | 0 |
| 3637 | 16 | 127 | 1.5 | 107 | 0 |
| | 4 | 136 | 0 | 129 | 0 |
| | 1 | 95 | 0 | 86 | 0 |
| 3700 | 16 | 143 | 4 | 159 | 4.5 |
| | 4 | 121 | 2.5 | 150 | 3.5 |
| | 1 | 104 | 0 | 141 | 1 |
| 3706 | 16 | 105 | 0.5 | 21 | 0 |
| | 4 | 109 | 0 | 64 | 0 |
| | 1 | 82 | 0 | 86 | 0 |

GROWTH REGULATING EFFECTS ON TWO SPECIES (continued)

| | | *Soja max* | | *Lycopersicum esculentum* | |
|---|---|---|---|---|---|
| Comp'd. No. | Rate oz/A | Pod Count① Percent in Comparison to Untreated Plants | Severity of Growth Regulating Effect② | Fruit Count Percent in Comparison to Untreated Plants | Severity of Growth Regulating Effect② |
| 3707 | 16 | 123 | 2 | 300 | 3 |
| | 4 | 150 | 1 | 150 | 1 |
| | 1 | 136 | 0 | 129 | 0 |
| 3709 | 16 | 109 c | 5.5 | 300 | 2.5 |
| | 4 | 145 | 4 | 364 | 1 |
| | 1 | 114 | 0.5 | 321 | 0.5 |
| 3710 | 16 | 167 a | 8.5 | 900 a | 8 |
| | 4 | 136 c | 5 | 700 b | 5.5 |
| | 1 | 143 | 3.5 | 700 | 4.5 |
| 3751 | 16 | 90 | 0 | 75 | 0 |
| | 4 | 93 | 0 | 300 | 0 |
| | 1 | 93 | 0 | 75 | 0 |
| 3752 | 16 | 183 | 8 | 225 | 9 |
| | 4 | 153 | 4.5 | 600 | 8.5 |
| | 1 | 161 | 2.5 | 975 | 5 |
| 3789 | 16 | 93 | 0.5 | 75 | 0.5 |
| | 4 | 117 | 0 | 75 | 0 |
| | 1 | 93 | 0 | 150 | 0 |
| 3792 | 16 | 145 | 8 | 525 | 9 |
| | 4 | 123 | 4 | 1050 | 7 |
| | 1 | 117 | 2 | 675 | 4.5 |
| 3812 | 16 | 106 | 3 | 392[1,2] | 1.5 |
| | 4 | 95 | 1 | 485[2] | 1.5 |
| | 1 | 106 | 0 | 369[2] | 1 |
| 3849 | 16 | 166 a | 7.5 | 254 d | 8.5 |
| | 4 | 166 c | 5 | 692 b | 6 |
| | 1 | 127 | 3 | 438 | 4.5 |
| 3850 | 16 | 113 | 2 | 277 | 3 |
| | 4 | 113 | 0 | 485 | 2.5 |
| | 1 | 120 | 0 | 369 | 1 |
| 3952 | 16 | 82 | 2 | 129 | 0 |
| | 4 | 105 | 0.5 | 129 | 0 |
| | 1 | 105 | 0 | 64 | 0 |
| 3977 | 16 | 137 | 5 | 1000 | 7 |
| | 4 | 119 | 1.5 | 550 | 2.5 |
| | 1 | 112 | 0 | 250 | 1 |
| 4062 | 16 | 133 a | 7 | 236 b | 4 |
| | 4 | 142 c | 5 | 139 | 1.5 |
| | 1 | 146 | 2.5 | 161 | 1.5 |
| 4120 | 16 | 100 a | 4.5 | 214 | 4.5 |
| | 4 | 142 c | 2.5 | 139 | 4 |
| | 1 | 117 | 1 | 129 | 2 |

GROWTH REGULATING EFFECTS ON TWO SPECIES (continued)

| | | *Soja max* | | *Lycopersicum esculentum* | |
|---|---|---|---|---|---|
| Comp'd. No. | Rate oz/A | Pod Count[1] Percent in Comparison to Untreated Plants | Severity of Growth Regulating Effect[2] | Fruit Count Percent in Comparison to Untreated Plants | Severity of Growth Regulating Effect[2] |
| 4121 | 16 | 158 | 3 | 129 | 1 |
| | 4 | 142 | 0.5 | 107 | 0.5 |
| | 1 | 121 | 0 | 86 | 0 |
| 4122 | 16 | 125 | 5 | 96 | 1.5 |
| | 4 | 117 | 2.5 | 96 | 1 |
| | 1 | 96 | 0.5 | 139 | 0 |
| 4123 | 16 | 104 | 0 | 139 | 0.5 |
| | 4 | 100 | 0 | 118 | 0 |
| | 1 | 113 | 0 | 150 | 0 |
| 4124 | 16 | 121 | 4.5 | 139 | 1 |
| | 4 | 113 | 0.5 | 129 | 0 |
| | 1 | 104 | 0 | 139 | 0 |
| 4125 | 16 | 113 | 1.5 | 129 | 0.5 |
| | 4 | 113 | 0 | 161 | 0.5 |
| | 1 | 88 | 0 | 107 | 0 |

[1]Check = 100

[2]Greenhouse rating on scale of 0, no effect to 10, total kill.

a injury
b malformed fruit
c smaller pods
d increased growth
e pear-shaped fruit

The information presented in tabular form herein will enable a worker in the art to make a selection from among the growth regulant compounds of the invention and to make some judgment with regard to application rates, depending upon the effect which is desired. It may be seen, for example, that total kills of some species of vegetation may occur at application rates as high as 5 to 10 lb. per acre, whereas beneficial effects may be observed on living plants at application rates of 1 lb. per acre or less.

The growth regulant compounds are usually applied in combination with inert carriers or diluents, as in aqueous sprays, granules and dust formulations in accordance with established practice in the art. An aqueous spray is usually prepared by mixing a wettable powder or emulsifiable concentrate formulation of a growth regulant with a relatively large amount of water to form a dispersion.

Wettable powders comprise intimate, finely divided mixtures of growth regulant compounds, inert solid carriers and surface active agents. The inert solid carrier is usually chosen from among the attapulgite clays, the kaolin clays, the montmorillonite clays, the diatomaceous earths, finely divided silica and purified silicates. Effective surfactants, which have wetting, penetrating and dispersing ability are usually present in a wettable powder formulation in proportions of from 0.5 to about 10 percent by weight. Among the surface active agents commonly used for this purpose are the sulfonated lignins, naphthalenesulfonates and condensed naphthalenesulfonates, alkylbenzenesulfonates, alkyl sulfates and non-ionic surfactants such as products of condensation of ethylene oxide with alkylphenols.

Emulsifiable concentrates of the growth regulant compounds comprise in each instance, a solution of growth regulant compound in a liquid carrier which is a mixture of water-immiscible solvent and surfactants, including emulsifiers. Useful solvents include aromatic hydrocarbon solvents such as the xylenes, alkylnaphthalenes, petroleum distillates, terpene solvents, ether-alcohols and organic ester solvents. Suitable emulsifiers, dispersing and wetting agents may be selected from the same classes of products which are employed in formulating wettable powders.

Usually, the growth regulators are applied by diluting with water agricultural compositions which desirably contain from 0.1 percent to 95 percent by weight of active compound and from 0.1 to 75 percent of a carrier or surfactant. However, direct application to plant seeds prior to planting may be accomplished in some instances by mixing powdered solid growth regulator with seed to obtain a substantially uniform coating which is very thin and comprises only one or two percent by weight or less, based on the weight of the seed. In most instances, however, a nonphytotoxic solvent, such as methanol is employed as a carrier to facilitate the uniform distribution of growth regulator on the surface of the seed.

When a compound is to be applied to the soil, as for a pre-emergence application, granular formulations are sometimes more convenient than sprays. A typical granular formation comprises the growth regulator compound dispersed on an inert carrier such as coarsely ground clay, or clay which has been converted to granules by treatment of a rolling bed of the powdered material with a small amount of liquid in a granulating drum. In the usual process for preparing granular formulations, a solution of the active compound is sprayed on the granules while they are being agitated in a suitable mixing apparatus, after which the granules are dried with a current of air during continued agitation.

## Claims

1. Compounds which have the structural formula:

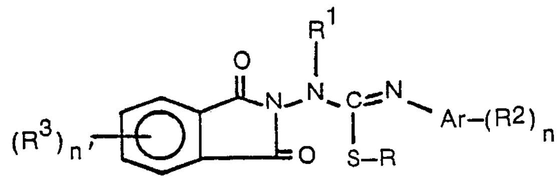

In which R is $C_1$ to $C_4$ alkyl, alkenyl or alkynyl to which may be attached phenyl, pyridyl, benzoyl, phenoxy, halophenoxy, halobenzoyl, N-phenylcarbamyl, N-ethyl-N-phenylcarbamyl, N-trifluoromethylthiadiazolylcarbamyl, ethoxycarbonyl, t.butylcarbonyl, methylthio, phenylcarbonyl, 4-Br-phenyl, 2,6-di-Cl-phenyl, or halo substituents,

$R^1$ is H, $C_1$ to $C_3$ alkyl, alkenyl or alkynyl to which may be attached phenyl, halophenyl or phenoxy groups, acetyl, ethoxycarbonyl, or R and $R^1$ together may be 1,4-butadienyl,

Ar is phenyl or benzoyl,

$R^2$ is $C_1$ to $C_4$ alkyl, alkoxy, diethylamino, phenoxy, benzyloxy, carbalkoxy, acetyl, methylenedioxy, trifluoromethyl, nitro, halo or cyano and n may be zero or an integer from 1 to 4 and when $n = 2$ $R^2$ may be a 3,4 propylene, with the provision that at least one position ortho to the point of attachment of a phenyl ring of the Ar structure must be unsubstituted,

$R^3$ is $C_1$—$C_4$ alkyl or halo and n' may be zero or an integer from 1 to 4, and hydrohalide salts of the said compounds.

2. The method of regulating the growth of plants comprising applying to the plants, the seed or the soil an effective amount of a compound as specified in Claim 1.

3. The method of regulating the growth of plants which comprises applying to the plants pre- or post-emergently an effective amount of a composition comprising from 0.1 percent to 95 weight percent of a compound of claim 1 in combination with from 0.1 to 75 weight percent of a carrier or surfactant.

4. The method of increasing fruit set of crop plants which comprises applying to the plant foliage an effective amount of a compound of claim 1 in combination with an inert carrier and a surfactant.

5. The method of claim 4 in which the crop plants are of the species *Lycopersicum esculentum*.

6. The method of claim 4 in which the crop plants are of the species *Soja max*.

7. 2-[1-Methyl-2-allyl-3-(3-fluorophenyl)isothioureido]-1H-isoindole-1,3-(2H)dione.

8. 2-(1-Methyl-2-propyl-3-phenylisothioureido)-1H-isoindole-1,3-(2H)dione.

9. 2-[1-Methyl-2-(4-bromobenzyl)-3-phenylisothioureido]-1H-isoindole-1,3-(2H)dione.

10. 2-(1-Methyl-2-pivalylmethyl-3-phenylisothioureido)-1H-isoindole-1,3-(2H)dione.

11. 2-(1,2-Dimethyl-3-phenylisothioureido)-1H-isoindole-1,3-(2H)dione hydroiodide.

12. 2-(1-Methyl-2-allyl-3-phenylisothioureido)-1H-isoindole-1,3-(2H)dione hydrobromide.

13. 2-(1-Methyl-2-benzyl-3-phenylisothioureido)-1H-isoindole-1,3-(2H)dione hydrobromide.

14. 2-(1-Allyl-2-methyl-3-phenylisothioureido)-1H-isoindole-1,3-(2H)dione.

15. 2-(1,2-Diallyl-3-phenylisothioureido)-1H-isoindole-1,3-(2H)dione.

16. 2-[1,2-Dimethyl-3-(3-methylphenyl)isothioureido]-1H-isoindole-1,3-(2H)dione.

17. 2-[1-Methyl-2(2-propynyl)-3-(3-fluorophenyl)isothioureido]-1H-isoindole-1,3-(2H)dione hydrobromide.

18. 2-[1,2-Dimethyl-3-(3-chlorophenyl)isothioureido]-1H-isoindole-1,3-(2H)dione.

19. 2-[1,2-Dimethyl-3-(3,4-dichlorophenyl)isothioureido]-1H-isoindole-1,3-(2H)dione.

20. 2-(1-Methyl-2-allyl-3-phenylisothioureido)-1H-isoindole-1,3-(2H)dione.
21. 2-(1,2-Dimethyl-3-phenylisothioureido)-1H-isoindole-1,3-(2H)dione.
22. 2-(1-Methyl-2-benzyl-3-phenylisothioureido)-1H-isoindole-1,3-(2H)dione.
23. 2-[1,2-Dimethyl-3-(3-fluorophenyl)isothioureido]-1H-isoindole-1,3-(2H)dione.
24. 2-(1-Methyl-2-ethyl-3-phenylisothioureido)-1H-isoindole-1,3-(2H)dione.
25. 2-[1-Methyl-2-butyl-3-(3,4-dichlorophenyl)isothioureido]-1H-isoindole-1,3-(2H)dione.
26. 2-[1-Methyl-2-methylthiomethyl-3-(3-chlorophenyl)isothioureido]-1H-isoindole-1,3-(2H)dione.
27. 2-(1,2-Dimethyl-3-phenylisothioureido)-4-methyl-1H-isoindole-1,3-(2H)dione.
28. 2-(1,2-Dimethyl-3-phenylisothioureido)-4-methyl-1H-isoindole-1,3-(2H)dione hydriodide.
29. 2-[1-Methyl-2-carbethoxymethyl-3-(4-fluorophenyl)isothioureido]-1H-isoindole-1,3-(2H)dione hydrobromide.
30. 2-[1-Methyl-2-carbethoxymethyl-3-(4-fluorophenyl)isothioureido]-1H-isoindole-1,3-(2H)dione.
31. 2-[1-Methyl-2-carbethoxymethyl-3-(3-fluorophenyl)isothioureido]-1H-isoindole-1,3-(2H)dione hydrobromide.
32. 2-(2-Ethyl-3-phenylisothioureido)-1H-isoindole-1,3-(2H)dione.
33. 2-(1-Allyl-2-ethyl-3-phenylisothioureido)-1H-isoindole-1,3-(2H)dione.
34. 2-[1,2-Dimethyl-3-(3-trifluoromethylphenyl)isothioureido]-1H-isoindole-1,3-(2H)dione.
35. 2-[1,2-Dimethyl-3-(4-chlorophenyl)isothioureido]-1H-isoindole-1,3-(2H)dione.
36. 2-[1-Methyl-2-benzyl-3-(4-chlorophenyl)isothioureido]-1H-isoindole-1,3-(2H)dione.
37. 2-[1-(2-Propynyl)2-benzyl-3-(4-chlorophenyl)isothioureido]-1H-isoindole-1,3-(2H)dione.
38. 2-[1-Methyl-2-(2,6-dichlorobenzyl)-3-phenylisothioureido]-1H-isoindole-1,3-(2H)dione.
39. 2-[1-Methyl-2-(4-methylbenzyl)-3-phenylisothioureido]-1H-isoindole-1,3-(2H)dione.
40. 2-(1-Allyl-2-propyl-3-phenylisothioureido)-1H-isoindole-1,3-(2H)dione.
41. 2-(1-Methyl-2-isopropyl-3-phenylisothioureido)-1H-isoindole-1,3-(2H)dione.
42. 2-[1,2-Dimethyl-3-(4-methoxyphenyl)isothioureido]-1H-isoindole-1,3-(2H)dione.
43. 2-[-2-Ethyl-1-methyl-3-(4-methoxyphenyl)isothioureido]-1H-isoindole-1,3-(2H)dione.
44. 2-[1,2-Dimethyl-3-(2-methylphenyl)isothioureido]-1H-isoindole-1,3-(2H)dione.
45. 2-[2-Ethyl-1-methyl-3-(2-methylphenyl)isothioureido]-1H-isoindole-1,3-(2H)dione.
46. 2-[1,2-Dimethyl-3-(2-chlorophenyl)isothioureido]-1H-isoindole-1,3-(2H)dione.
47. 2-[1,2-Dimethyl-3-(4-fluorophenyl)isothioureido]-1H-isoindole-1,3-(2H)dione.
48. 2-[2-Ethyl-1-methyl-3-(2-chlorophenyl)isothioureido]-1H-isoindole-1,3-(2H)dione.
49. 2-[2-Ethyl-1-methyl-3-(4-fluorophenyl)isothioureido]-1H-isoindole-1,3-(2H)dione.
50. 2-(1-Allyl-2-isopropyl-3-phenylisothioureido-1H-isoindole-1,3-(2H)dione.
51. 2-(2-Butyl-1-methyl-3-phenylisothioureido)-1H-isoindole-1,3-(2H)dione.
52. 2-(1-Allyl-2-butyl-3-phenylisothioureido)-1H-isoindole-1,3-(2H)dione.
53. 2-(2-Isobutyl-1-methyl-3-phenylisothioureido)-1H-isoindole-1,3-(2H)dione.
54. 2-[1,2-Dimethyl-3-(4-chloro-3-trifluoromethylphenyl)isothioureido-1H-isoindole-1,3-(2H)dione.
55. 2-[1,2-Dimethyl-3(2-trifluoromethylphenyl)isothioureido]-1H-isoindole-1,3-(2H)dione.
56. 2-{2-[2-(2,4-Dichlorophenoxy)ethyl]-1-methyl-3-phenylisothioureido}-1H-isoindole-1,3-(2H)dione.
57. 2-[1,2-Dimethyl-3-(3-methoxyphenyl)isothioureido]-1H-isoindole-1,3-(2H)dione.
58. 2-[1,2-Dimethyl-3-(3-methylthiophenyl)isothioureido]-1H-isoindole-1,3-(2H)dione.
59. 2-[1,2-Dimethyl-3-(4-chloro-2-methylphenyl)isothioureido]-1H-isoindole-1,3-(2H)dione.
60. 2-[1,2-Dimethyl-3-(2,4-dichlorophenyl)isothioureido]-1H-isoindole-1,3-(2H)dione.
61. 2-[1,2-Dimethyl-3-(4-ethoxyphenyl)isothioureido]-1H-isoindole-1,3-(2H)dione.
62. 2-[1,2-Dimethyl-3-(2-fluorophenyl)isothioureido]-1H-isoindole-1,3-(2H)dione.
63. 2-[2-Ethyl-1-methyl-3-(2,4-dichlorophenyl)isothioureido]-1H-isoindole-1,3-(2H)dione.
64. 2-[2-Ethyl-1-methyl-3-(4-ethoxyphenyl)isothioureido]-1H-isoindole-1,3-(2H)dione.
65. 2-[2-Allyl-1-methyl-3-(4-trifluoromethylphenyl)isothioureido]-1H-isoindole-1,3-(2H)dione.
66. 2-{2-[2-(2,4-Dichlorophenoxy)ethyl]-1-methyl-3-(2-fluorophenyl)isothioureido}-1H-isoindole-1,3-(2H)dione.
67. 2-[2-(1-Benzoyl)ethyl-1-methyl-3-(4-methoxyphenyl)-isothioureido]-1H-isoindole-1,3-(2H)dione.
68. 2-[2-(4-Fluorobenzoylmethyl)-1-methyl-3-(2-methylphenyl)isothioureido]-1H-isoindole-1,3-(2H)dione.
69. 2-[2-(4-Fluorobenzoylmethyl)-1-methyl-3-(2-fluorophenyl)isothioureido]-1H-isoindole-1,3-(2H)dione.
70. 2-(2-Carboxymethyl-1-methyl-3-phenylisothioureido)-1H-isoindole-1,3-(2H)dione hydrobromide.
71. 2-[1-(3-Chlorobenzyl)-2-methyl-3-phenylisothioureido]-1H-isoindole-1,3-(2H)dione.
72. 2-[2-(3-Phenyl-2-propenyl)-3-(3-fluorophenyl)isothioureido]-1H-isoindole-1,3-(2H)dione.

73. 2{2-[2-(N-Ethyl-N-phenylcarbamyl)ethyl]-1-methyl-3-(3-fluorophenyl)isothioureido}-1H-isoindole-1,3-(2H)dione.

74. 2-[1-Methyl-2-(3,4,4-trifluoro-3-butenyl)-3-(3-fluorophenyl)isothioureido]-1H-isoindole-1,3-(2H)dione.

75. 2-[1-Methyl-2-(3,4,4-trifluoro-3-butenyl)-3-phenylisothioureido]-1H-isoindole-1,3-(2H)dione.

76. 2-[1-Methyl-2-(4-pyridylmethyl)-3-phenylisothioureido]-1H-isoindole-1,3-(2H)dione.

77. 2-[1-Methyl-2-(3-pyridylmethyl)-3-phenylisothioureido]-1H-isoindole-1,3-(2H)dione.

78. 2-[1-Methyl-2-(2-pyridylmethyl)-3-(3-fluorophenyl)isothioureido]-1H-isoindole-1,3-(2H)dione.

79. 2-[1-Methyl-2-(3-pyridylmethyl)-3-(3-fluorophenyl)isothioureido]-1H-isoindole-1,3-(2H)dione.

80. 2-[1,2-Dimethyl-3-(3-methylbenzoyl)isothioureido]-1H-isoindole-1,3-(2H)dione.

81. The method of manufacturing compounds according to claim 1 comprising reacting a compound of the formula:

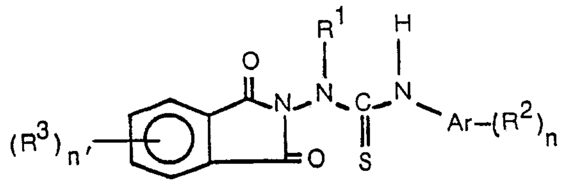

in a non-reactive polar organic solvent with a compound of the formula R—X in which X is a leaving group, in particular a chlorine, bromine, iodine or sulfate substituent.

82. The method of claim 81 in which the compounds are reacted at room temperature in the presence of a strongly basic compound which acts as an acid acceptor.

**Revendications**

1. Composés qui ont la formule de structure:

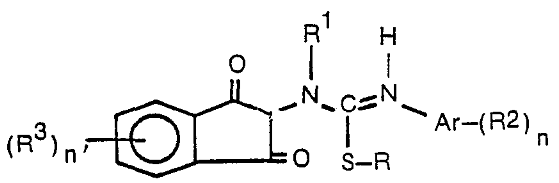

[dans laquelle R représente un groupe alkyle en $C_1$—$C_4$, alcényle ou alcynyle sur lequel peuvent être fixés des substituants phényle, pyridyle, benzoyle, phénoxy, halogénophénoxy, halogénobenzoyle, N-phénylcarbamyle, N-éthyl-N-phényl-carbamyle, N-trifluorométhylthiadiazolylcarbamyle, éthoxycarbonyle, t.butylcarbonyle, méthylthio, phénylcarbonyle, 4-bromo-phényle, 2,6-dichlorophényle ou halogéno;

$R^1$ représente H, un groupe alkyle en $C_1$ à $C_3$, alcényle ou alcynyle (sur lequel peuvent être fixés des groupes phényle, halogénophényle ou phénoxy), acétyle, éthoxycarbonyle, ou bien R et $R^1$ pris ensemble peuvent représenter un groupe 1,4-butadiényle,

Ar est un groupe phényle ou benzoyle;

$R^2$ représente un groupe alkyle en $C_1$ à $C_4$, alcoxy, diéthylamino, phénoxy, benzyloxy, carbalcoxy, acétyle, méthylènedioxy, trifluorométhyle, nitro, halogéno ou cyano et n peut être nul ou être un nombre entier valant 1 à 4 et, lorsque n vaut 2, $R^2$ peut être un groupe 2,3-propylène, étant bien entendu qu'au moins une position ortho par rapport au point de fixation d'un noyau phényle de la structure Ar doit être non substitué,

$R^3$ représente un groupe alkyle en $C_1$—$C_4$ ou halogéno, et n' peut être nul ou être un nombre entier valant 1 à 4] et sels qui sont les halogenhydrates desdits composés.

2. Procédé pour réguler la croissance de plantes, comprenant l'application aux plantes, aux graines ou à la terre, d'une quantité efficace d'un composé tel que spécifié à la revendication 1.

3. Procédé pour réguler la croissance de plantes, qui comprend l'application aux plantes, avant ou après la levée, d'une quantité efficace d'une composition comprenant de 0,1% à 95% en poids d'un composé selon la revendication 1, en combinaison avec de 0,1 à 75% en poids d'un support, véhicule ou surfactif.

4. Procédé pour augmenter la nouaison de plantes cultivées, qui comprend l'application au feuillage des plantes d'une quantité efficace d'un composé selon la revendication 1, en combinaison avec un support ou véhicule inerte et avec un surfactif.

5. Procédé selon la revendication 4, dans lequel les plantes cultivées appartiennent à l'espèce *Lycopersicum esculentum.*

6. Procédé selon la revendication 4, dans lequel les plantes cultivées appartiennent à l'espèce *Soja max.*

7. La 2-[1-méthyl-2-allyl-3-(3-fluorophényl)isothiouréido]-1H-isoindole-1,3-(2H)dione.

8. La 2-[1-méthyl-2-propyl-3-phénylisothiouréido]-1H-isoindole-1,3-(2H)dione.

9. La 2-[1-méthyl-2-(4-bromobenzyl)-3-phénylisothiouréido]-1H-isoindole-1,3-(2H)dione.

10. La 2-[1-méthyl-2-pivalylméthyl-3-phénylisothiouréido]-1H-isoindole-1,3-(2H)dione.

11. Le iodhydrate de la 2-[1,2-diméthyl-3-phénylisothiouréido]-1H-isoindole-1,3-(2H)dione.

12. Le bromhydrate de la 2-[méthyl-2-allyl-3-phénylisothiouréido]-1H-isoindole-1,3-(2H)dione.

13. Le bromhydrate de la 2-[1-methyl-2-benzyl-3-phénylisothiouréido]-1H-isoindole-1,3-(2H)dione.

14. La 2-[1-allyl-2-méthyl-3-phénylisothiouréido]-1H-isoindole-1,3-(2H)dione.

15. La 2-[1,2-diallyl-3-phénylisothiouréido]-1H-isoindole-1,3-(2H)dione.

16. La 2-[1,2-diméthyl-3-(3-méthylphényl)isothiouréido]-1H-isoindole-1,3-(2H)dione.

17. Le bromhydrate de la 2-[1-méthyl-2(2-propynyl)-3-3(3-fluorophényl)isothiouréido]-1H-isoindole-1,3-(2H)dione.

18. La 2-[1,2-diméthyl-3-(3-chlorophényl)isothiouréido]-1H-isoindole-1,3-(2H)dione.

19. La 2-[1,2-diméthyl-3-(3,4-dichlorophényl)isothiouréido]-1H-isoindole-1,3-(2H)dione.

20. La 2-(1-méthyl-2-allyl-3-phénylisothiouréido)-1H-isoindole-1,3-(2H)dione.

21. La 2-(1,2-diméthyl-3-phénylisothiouréido)-1H-isoindole-1,3-(2H)dione.

22. La 2-(1-méthyl-2-benzyl-3-phénylisothiouréido)-1H-isoindole-1,3-(2H)dione.

23. La 2-[1,2-diméthyl-3-(3-fluorophényl)isothiouréido]-1H-isoindole-1,3-(2H)dione.

24. La 2-[1-méthyl-2-éthyl-3-phénylisothiouréido]-1H-isoindole-1,3-(2H)dione.

25. La 2-[1-méthyl-2-butyl-3-(3,4-dichlorophényl)isothiouréido]-1H-isoindole-1,3-(2H)dione.

26. La 2-[1-méthyl-2-méthylthiométhyl-3-(3-chlorophényl)isothiouréido]-1H-isoindole-1,3-(2H)dione.

27. La 2-[1,2-diméthyl-3-phénylisothiouréido]-4-méthyl-1H-isoindole-1,3-(2H)dione.

28. Le iodhydrate de la 2-[1,2-diméthyl-3-phénylisothiouréido]-4-méthyl-1H-isoindole-1,3-(2H)dione.

29. Le bromhydrate de la 2-[1-méthyl-2-carbéthoxyméthyl-3-(4-fluorophényl)isothiouréido]-1H-isoindole-1,3-(2H)dione.

30. La 2-[1-méthyl-2-carbéthoxyméthyl-3-(4-fluorophényl)isothiouréido]-1H-isoindole-1,3-(2H)dione.

31. Le bromhydrate de la 2-[1-méthyl-2-carbéthoxyméthyl-3-(3-fluorophényl)isothiouréido]-1H-isoindole-1,3-(2H)dione.

32. La 2-[2-éthyl-3-phénylisothiouréido]-1H-isoindole-1,3-(2H)dione.

33. La 2-[1-allyl-2-éthyl-3-phénylisothiouréido]-1H-isoindole-1,3-(2H)dione.

34. La 2-[1,2-diméthyl-3-(3-trifluorométhylphényl)isothiouréido]-1H-isoindole-1,3-(2H)dione.

35. La 2-[1,2-diméthyl-3-(4-chlorophényl)isothiouréido]-1H-isoindole-1,3-(2H)dione.

36. La 2-[1-méthyl-2-benzyl-3-(4-chlorophényl)isothiouréido]-1H-isoindole-1,3-(2H)dione.

37. La 2-[1-(2-propynyl)2-benzyl-3-(4-chlorophenyl)isothiouréido]-1H-isoindole-1,3-(2H)dione.

38. La 2-[1-méthyl-2-(2,6-dichlorobenzyl)-3-phénylisothiouréido]-1H-isoindole-1,3-(2H)dione.

39. La 2-[1-méthyl-2-(4-méthylbenzyl)-3-phénylisothiouréido]-1H-isoindole-1,3-(2H)dione.

40. La 2-[1-allyl-2-propyl-3-phényl-isothiouréido]-1H-isoindole-1,3-(2H)dione.

41. La 2-[1-méthyl-2-isopropyl-3-phényl-isothiouréido]-1H-isoindole-1,3-(2H)dione.

42. La 2-[1,2-diméthyl-3-(4-méthoxyphényl)isothiouréido]-1H-isoindole-1,3-(2H)dione.

43. La 2-[2-éthyl-1-méthyl-3-(4-méthoxyphényl)isothiouréido]-1H-isoindole-1,3-(2H)dione.

44. La 2-[1,2-diméthyl-3-(2-méthylphényl)isothiouréido]-1H-isoindole-1,3-(2H)dione.

45. La 2-[2-éthyl-1-méthyl-3-(2-méthylphényl)isothiouréido]-1H-isoindole-1,3-(2H)dione.

46. La 2-[1,2-diméthyl-3-(2-chlorophényl)isothiouréido]-1H-isoindole-1,3-(2H)dione.

47. La 2-[1,2-diméthyl-3-(4-fluorophényl)isothiouréido]-1H-isoindole-1,3-(2H-dione.

48. La 2-[2-éthyl-1-méthyl-3-(2-chlorophényl)isothiouréido]-1H-isoindole-1,3-(2H)dione.

49. La 2-[2-éthyl-1-méthyl-3-(4-fluorophényl)isothiouréido]-1H-isoindole-1,3-(2H)dione.

50. La 2-(1-allyl-2-isopropyl-3-phénylisothiouréido)-1H-isoindole-1,3-(2H)dione.

51. La 2-(2-butyl-1-méthyl-3-phénylisothiouréido)-1H-isoindole-1,3-(2H)dione.

52. La 2-(1-allyl-2-butyl-3-phénylisothiouréido)-1H-isoindole-1,3-(2H)dione.

53. La 2-(2-isobutyl-1-méthyl-3-phénylisothiouréido)-1H-isoindole-1,3-(2H)dione.

54. La 2-[1,2-diméthyl-3-(4-chloro-3-trifluorométhylphényl)isothiouréido]-1H-isoindole-1,3-(2H)dione.

55. La 2-(1,2-diméthyl-3(2-trifluorométhylphényl)isothiouréido]-1H-isoindole-1,3-(2H)dione.

56. La 2-{2-(2,4-dichlorophénoxy)éthyl)-1-méthyl-3-phénylisothiouréido}-1H-isoindole-1,3-(2H)dione.

57. La 2-[1,2-diméthyl-3-(3-méthoxyphényl)isothiouréido]-1H-isoindole-1,3-(2H)dione.

58. La 2-[1,2-diméthyl-3-(3-méthylthiophénylisothiouréido]-1H-isoindole-1,3-(2H)dione.

59. La 2-[1,2-diméthyl-3-(4-chloro-2-méthylphényl)isothiouréido]-1H-isoindole-1,3-(2H)dione.
60. La 2-[1,2-diméthyl-3-(2,4-dichlorophényl)isothiouréido]-1H-isoindole-1,3-(2H)dione.
61. La 2-[1,2-diméthyl-3-(4-éthoxyphényl)isothiouréido]-1H-isoindole-1,3-(2H)dione.
62. La 2-[1,2-diméthyl-3-(2-fluorophényl)isothiouréido]-1H-isoindole-1,3-(2H)dione.
63. La 2-[2-éthyl-1-méthyl-3-(2,4-dichlorophényl)isothiouréido]-1H-isoindole-1,3-(2H)dione.
64. La 2-[2-éthyl-1-méthyl-3-(4-éthoxyphényl)isothiouréido]-1H-isoindole-1,3-(2H)dione.
65. La 2-[2-allyl-1-méthyl-3-(4-trifluorométhylphényl)isothiouréido]-1H-isoindole-1,3-(2H)dione.
66. La 2-{2-[2-(2,4-dichlorophénoxy)éthyl]-1-méthyl-3-(2-fluorophényl)isothiouréido}-1H-isoindole-1,3-(2H)dione.
67. La 2-[2-(1-benzoyl)éthyl-1-méthyl-3-(4-méthoxyphényl)isothiouréido]-1H-isoindole-1,3-(2H)dione.
68. La 2-[2-(4-fluorobenzoylméthyl)-1-méthyl-3-(2-méthylphényl)isothiouréido]-1H-isoindole-1,3-(2H)dione.
69. La 2-[2-(4-fluorobenzoylméthyl)-1-méthyl-3-(2-fluorophényl)isothiouréido]-1H-isoindole-1,3-(2H)dione.
70. Le bromhydrate de la 2-(2-carboxyméthyl-1-méthyl-3-phényl-isothiouréido)-1H-isoindole-1,3-(2H)dione.
71. La 2-[1-(3-chlorobenzyl)-2-méthyl-3-phénylisothiouréido]-1H-isoindole-1,3-(2H)dione.
72. La 2-[2-(3-phényl-2-propényl)-3-(3-fluorophényl)isothiouréido]-1H-isoindole-1,3-(2H)dione.
73. La 2{2-[2-(N-éthyl-N-phénylcarbamyl)éthyl]-1-méthyl-3-(3-fluorophényl)isothiouréido}-1H-isoindole-1,3-(2H)dione.
74. La 2-[1-méthyl-2-(3,4,4-trifluoro-3-butényl)-3-(3-fluorophényl)isothiouréido]-1H-isoindole-1,3-(2H)dione.
75. La 2-[1-méthyl-2-(3,4,4-trifluoro-3-butényl-3-phénylisothiouréido]-1H-isoindole-1,3-(2H)dione.
76. La 2-[1-méthyl-2-(4-pyridylméthyl)-3-phénylisothiouréido]-1H-isoindole-1,3-(2H)dione.
77. La 2-[1-méthyl-2-(3-pyridylméthyl)-3-phénylisothiouréido]-1H-isoindole-1,3-(2H)dione.
78. La 2-[1-méthyl-2-(2-pyridylméthyl)-3-(3-fluorophényl)isothiouréido]-1H-isoindole-1,3-(2H)dione.
79. La 2-[1-méthyl-2-(3-pyridylméthyl)-3-(3-fluorophényl)isothiouréido]-1H-isoindole-1,3-(2H)dione.
80. La 2-[1,2-diméthyl-3-(3-méthylbenzoyl)isothiouréido]-1H-isoindole-1,3-(2H)dione.
81. Procédé de fabrication de composés selon la revendication 1, comprenant la réaction d'un composé de formule:

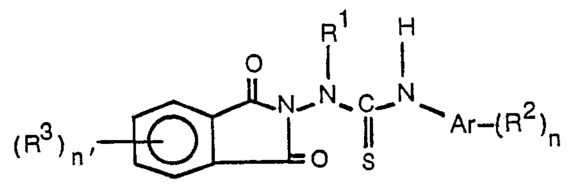

dans un solvant organique polaire non réactif, avec un composé de formule R—X, dans laquelle X est un groupe qui se sépare, en particulier un substituant chlore, brome, iode ou sulfate.
82. Procédé selon la revendication 81, dans lequel on fait réagir les composés à la température ambiante en présence d'un composé fortement basique jouant le rôle d'un accepteur d'acide.

**Patentansprüche**

1. Verbindung der Strukturformel

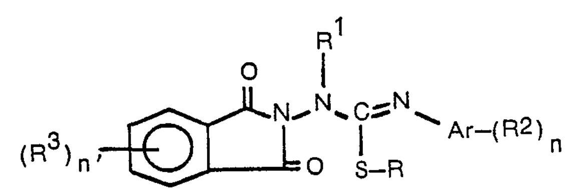

worin bedeuten

R $C_1$—$C_4$ Alkyl, Alkenyl oder Alkinyl, an die gebunden sein kann Phenyl, Pyridyl, Benzoyl, Phenoxy, Halogenphenoxy, Halogenbenzoyl, N-Phenylcarbamyl, N-Ethyl-N-phenyl-carbamyl, N-Trifluormethylthiadiazolylcarbamyl, Ethoxycarbonyl, tert.-Butylcarbonyl, Methylthio, Phenylcarbonyl, 4-Br-phenyl, 2,6-Di-Cl-phenyl oder Halogensubstituenten;

$R^1$ Wasserstoff, $C_1$—$C_3$ Alkyl, Alkenyl oder Alkinyl, an die gebunden sein kann Phenyl, Halogenphenyl- oder Phenoxy-Gruppen, Acetyl, Ethoxycarbonyl oder R und $R^1$ zusammen 1,4-Butadienyl;

Ar Phenyl oder Benzoyl;

$R^2$ $C_1$—$C_4$ Alkyl, Alkoxy, Diethylamino, Phenoxy, Benzyloxy, Carbalkoxy, Acetyl, Methylendioxy, Trifluormethyl, Nitro, Halogen oder Cyan und n = 0 oder eine ganze Zahl von 1 bis 4 und wenn n = 2 ist, kann $R^2$ sein ein 3,4-Propylen, wobei mindestens eine ortho-Stellung zu der Bindung eines Phenylringes oder der Ar-Struktur unsubstituiert sein muß;

$R^3$ $C_1$—$C_4$ Alkyl oder Halogen und n' = 0 oder eine ganze Zahl von 1 bis 4, und Halogenwasserstoffsalze dieser Verbindungen.

2. Verfahren zur Regulation des Pflanzenwachstums, gekennzeichnet dadurch, daß man auf die Pflanzen, die Saat oder das Erdreich eine wirksame Menge einer wie in Anspruch 1 angegebenen Verbindung aufbringt.

3. Verfahren zur Regulierung des Pflanzenwachstums, gekennzeichnet dadurch, daß man auf die Pflanzen vor oder nach dem Auflaufen eine wirksame Menge einer Formulierung, enthaltend 0,1 bis 95 Gew.-% einer in Anspruch 1 angegebenen Verbindung in Kombination mit 0,1 bis 75 Gew.-% eines Trägers oder oberflächenaktiven Mittels, aufbringt.

4. Verfahren zur Erhöhung der Fruchtmenge bei Erntepflanzen, gekennzeichnet, dadurch, daß man auf das Blattwerk eine wirksame Menge einer in Anspruch 1 angegebenen Verbindung in Kombination mit einem inerten Träger und einem oberflächenaktiven Mittel aufbringt.

5. Verfahren nach Anspruch 4, gekennzeichnet dadurch, daß die Erntepflanzen zu der Art Lycopersicum esculentum gehören.

6. Verfahren nach Anspruch 4, gekennzeichnet dadurch, daß die Erntepflanzen zu der Art Soja max. gehören.

7. 2-[1-Methyl-2-allyl-3-(3-fluor-phenyl)isothioureido]-1H-isoindol-1,3-(2H)dion.

8. 2-(1-Methyl-2-propyl-3-phenylisothioureido)-1H-isoindol-1,3-(2H)dion.

9. 2-[1-Methyl-2-(4-brom-benzyl)-3-phenylisothioureido]-1H-isoindol-1,3-(2H)dion.

10. 2-(1-Methyl-2-pivalylmethyl-3-phenylisothioureido)-1H-isoindol-1,3-(2H)dion.

11. 2-(1,2-Dimethyl-3-phenylisothioureido)-1H-isoindol-1,3-(2H)dion-hydrojodid.

12. 2-(1-Methyl-2-allyl-3-phenylisothioureido)-1H-isoindol-1,3-(2H)dion-hydrobromid.

13. 2-(1-Methyl-2-benzyl-3-phenylisothioureido)-1H-isoindol-1,3-(2H)dion-hydrobromid.

14. 2-(1-Allyl-2-methyl-3-phenylisothioureido)-1H-isoindol-1,3-(2H)dion.

15. 2-(1,2-Diallyl-3-phenylisothioureido)-1H-isoindol-1,3-(2H)dion.

16. 2-[1,2-Dimethyl-3-(3-methylphenyl)isothioureido]-1H-isoindol-1,3-(2H)dion.

17. 2-[1-Methyl-2(2-propynyl)-3-(3-fluor-phenyl)isothioureido]-1H-isoindol-1,3-(2H)dion-hydrobromid.

18. 2-[1,2-Dimethyl-3-(3-chlor-phenyl)isothioureido]-1H-isoindol-1,3-(2H)dion.

19. 2-[1,2-Dimethyl-3-(3,4-dichlor-phenyl)isothioureido]-1H-isoindol-1,3-(2H)dion.

20. 2-(1-Methyl-2-allyl-3-phenylisothioureido)-1H-isoindol-1,3-(2H)dion.

21. 2-(1,2-Dimethyl-3-phenylisothioureido)-1H-isoindol-1,3-(2H)dion.

22. 2-(1-Methyl-2-benzyl-3-phenylisothioureido)-1H-isoindol-1,3-(2H)dion.

23. 2-[1,2-Dimethyl-3-(3-fluor-phenyl)isothioureido]-1H-isoindol-1,3-(2H)dion.

24. 2-(1-Methyl-2-ethyl-3-phenylisothioureido)-1H-isoindol-1,3-(2H)dion.

25. 2-[1-Methyl-2-butyl-3-(3,4-dichlor-phenyl)isothioureido]-1H-isoindol-1,3-(2H)dion.

26. 2-[1-Methyl-2-methylthiomethyl-3-(3-chlor-phenyl)isothioureido]-1H-isoindol-1,3-(2H)dion.

27. 2-(1,2-Dimethyl-3-phenylisothioureido)-4-methyl-1H-isoindol-1,3-(2H)dion.

28. 2-(1,2-Dimethyl-3-phenylisothioureido)-4-methyl-1H-isoindol-1,3-(2H)dion-hydrojodid.

29. 2-[1-Methyl-2-carbethoxymethyl-3-(4-fluor-phenyl)isothioureido]-1H-isoindol-1,3-(2H)dion-hydrobromid.

30. 2-[1-Methyl-2-carbethoxymethyl-3-(4-fluor-phenyl)isothioureido]-1H-isoindol-1,3-(2H)dion.

31. 2-[1-Methyl-2-carbethoxymethyl-3-(3-fluor-phenyl)isothioureido]-1H-isoindol-1,3-(2H)dion hydrobromid.

32. 2-(2-äthyl-3-phenylisothioureido)-1H-isoindol-1,3-(2H)dion.

33. 2-(1-Allyl-2-äthyl-3-phenylisothioureido)-1H-isoindol-1,3-(2H)dion.

34. 2-[1,2-Dimethyl-3-(3-trifluor-methylphenyl)isothioureido]-1H-isoindol-1,3-(2H)dion.

35. 2-[1,2-Dimethyl-3-(4-chlor-phenyl)isothioureido]-1H-isoindol-1,3-(2H)dion.

36. 2-[1-Methyl-2-benzyl-3-(4-chlor-phenyl)isothioureido]-1H-isoindol-1,3-(2H)dion.

37. 2-[1-(2-Propynyl)2-benzyl-3-(4-chlor-phenyl)isothioureido]-1H-isoindol-1,3-(2H)dion.

38. 2-[1-Methyl-2-(2,6-dichlor-benzyl)-3-phenylisothioureido]-1H-isoindol-1,3-(2H)dion.

39. 2-[1-Methyl-2-(4-methylbenzyl)-3-phenylisothioureido]-1H-isoindol-1,3-(2H)dion.

40. 2-(1-Allyl-2-propyl-3-phenylisothioureido)-1H-isoindol-1,3-(2H)dion.

41. 2-(1-Methyl-2-isopropyl-3-phenylisothioureido)-1H-isoindol-1,3-(2H)dion.

42. 2-[1,2-Dimethyl-3-(4-methoxyphenyl)isothioureido]-1H-isoindol-1,3-(2H)dion.

43. 2-[-2-Äthyl-1-methyl-3-(4-methoxyphenyl)isothioureido]-1H-isoindol-1,3-(2H)dion.

44. 2-[1,2-Dimethyl-3-(2-methylphenyl)isothioureido]-1H-isoindol-1,3-(2H)dion.

45. 2-[2-Äthyl-1-methyl-3-(2-methylphenyl)isothioureido]-1H-isoindol-1,3-(2H)dion.

46. 2-[1,2-Dimethyl-3-(2-chlor-phenyl)isothioureido]-1H-isoindol-1,3-(2H)dion.

47. 2-[1,2-Dimethyl-3-(4-fluor-phenyl)isothioureido]-1H-isoindol-1,3-(2H)dion.

48. 2-[2-Äthyl-1-methyl-3-(2-chlor-phenyl)isothioureido]-1H-isoindol-1,3-(2H)dion.

49. 2-[2-Äthyl-1-methyl-3-(4-fluor-phenyl)isothioureido]-1H-isoindol-1,3-(2H)dion.
50. 2-(1-Allyl-2-isopropyl-3-phenylisothioureido)-1H-isoindol-1,3-(2H)dion.
51. 2-(2-Butyl-1-methyl-3-phenylisothioureido)-1H-isoindol-1,3-(2H)dion.
52. 2-(1-Allyl-2-butyl-3-phenylisothioureido)-1H-isoindol-1,3-(2H)dion.
53. 2-(2-Isobutyl-1-methyl-3-phenylisothioureido)-1H-isoindol-1,3-(2H)dion.
54. 2-[1,2-Dimethyl-3-(4-chlor-3-trifluor-methylphenyl)isothioureido]-1H-isoindol-1,3-(2H)dion.
55. 2-[1,2-Dimethyl-3(2-trifluor-methylphenyl)isothioureido]-1H-isoindol-1,3-(2H)dion.
56. 2-{2-[2-(2,4-Dichlor-phenoxy)äthyl]-1-methyl-3-phenylisothioureido}-1H-isoindol-1,3-(2H)dion.
57. 2-[1,2-Dimethyl-3-(3-methoxyphenyl)isothioureido]-1H-isoindol-1,3-(2H)dion.
58. 2-[1,2-Dimethyl-3-(3-methylthiophenyl)isothioureido]-1H-isoindol-1,3-(2H)dion.
59. 2-[1,2-Dimethyl-3-(4-chlor-2-methylphenyl)isothioureido]-1H-isoindol-1,3-(2H)dion.
60. 2-[1,2-Dimethyl-3-(2,4-dichlor-phenyl)isothioureido]-1H-isoindol-1,3-(2H)dion.
61. 2-[1,2-Dimethyl-3-(4-äthoxyphenyl)isothioureido]-1H-isoindol-1,3-(2H)dion.
62. 2-[1,2-Dimethyl-3-(2-fluor-phenyl)isothioureido]-1H-isoindol-1,3-(2H)dion.
63. 2-[2-Äthyl-1-methyl-3-(2,4-dichlor-phenyl)isothioureido]-1H-isoindol-1,3-(2H)dion.
64. 2-[2-Äthyl-1-methyl-3-(4-äthoxyphenyl)isothioureido]-1H-isoindol-1,3-(2H)dion.
65. 2-[2-Allyl-1-methyl-3-(4-trifluor-methylphenyl)isothioureido]-1H-isoindol-1,3-(2H)dion.
66. 2-{2-[2-(2,4-Dichlor-phenoxy)äthyl]-1-methyl-3-(2-fluor-phenyl)isothioureido}-1H-isoindol-1,3-(2H)dion.
67. 2-[2-(1-Benzoyl)äthyl-1-methyl-3-(4-methoxyphenyl)isothioureido]-1H-isoindol-1,3-(2H)dion.
68. 2-[2-(4-Fluor-benzoylmethyl)-1-methyl-3-(2-methylphenyl)isothioureido]-1H-isoindol-1,3-(2H)dion.
69. 2-[2-(4-Fluor-benzoylmethyl)-1-methyl-3-(2-fluor-phenyl)isothioureido]-1H-isoindol-1,3-(2H)dion.
70. 2-(2-Carboxymethyl-1-methyl-3-phenylisothioureido)-1H-isoindol-1,3-(2H)dion hydrobromid.
71. 2-[1-(3-Chlor-benzyl)-2-methyl-3-phenylisothioureido]-1H-isoindol-1,3-(2H)dion.
72. 2-[2-(3-Phenyl-2-propenyl)-3-(3-fluor-phenyl)isothioureido]-1H-isoindol-1,3-(2H)dion.
73. 2{2-[2-(N-Äthyl-N-phenylcarbamyl)äthyl]-1-methyl-3-(3-fluor-phenyl)isothioureido}-1H-isoindol-1,3-(2H)dion.
74. 2-[1-Methyl-2-(3,4,4-trifluor-3-butenyl)-3-(3-fluor-phenyl)isothioureido]-1H-isoindol-1,3-(2H)dion.
75. 2-[1-Methyl-2-(3,4,4-trifluor-3-butenyl)-3-phenylisothioureido]-1H-isoindol-1,3-(2H)dion.
76. 2-[1-Methyl-2-(4-pyridylmethyl)-3-phenylisothioureido]-1H-isoindol-1,3-(2H)dion.
77. 2-[1-Methyl-2-(3-pyridylmethyl)-3-phenylisothioureido]-1H-isoindol-1,3-(2H)dion.
78. 2-[1-Methyl-2-(2-pyridylmethyl)-3-(3-fluor-phenyl)isothioureido]-1H-isoindol-1,3-(2H)dion.
79. 2-[1-Methyl-2-(3-pyridylmethyl)-3-(3-fluor-phenyl)isothioureido]-1H-isoindol-1,3-(2H)dion.
80. 2-[1,2-Dimethyl-3-(3-methylbenzoyl)isothioureido]-1H-isoindol-1,3-(2H)dion.
81. Verfahren zur Herstellung von Verbindungen gemäß Anspruch 1, dadurch gekennzeichnet, daß man eine Verbindung der Formel

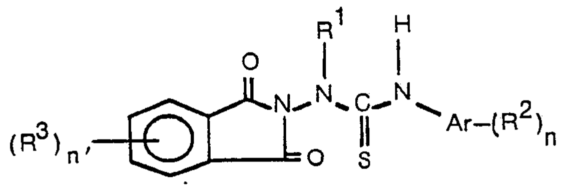

in einem nichtreaktiven polaren organischen Lösungsmittel mit einer Verbindung der Formel R—X umsetzt, in der X ein abspaltbarer Rest, insbesondere ein Chlor-, Brom-, Jod- oder Sulfatsubstituent ist.
82. Verfahren nach Anspruch 81, dadurch gekennzeichnet, daß die Verbindungen bei Raumtemperatur in Gegenwart einer stark basischen Verbindung, die als Säureakzeptor wirkt, zur Umsetzung gebracht werden.